(19) Europäisches Patentamt
European Patent Office
Office européen des brevets

(11) Numéro de publication: **0 112 776**
**B1**

(12) # FASCICULE DE BREVET EUROPÉEN

(45) Date de publication du fascicule du brevet: **22.07.87**

(21) Numéro de dépôt: **83402501.7**

(22) Date de dépôt: **21.12.83**

(51) Int. Cl.⁴: **A 61 K 31/47**, A 61 K 31/505, A 61 K 31/165, C 07 D 215/52, C 07 D 239/72, C 07 D 217/26, C 07 D 401/04, C 07 D 417/04, C 07 D 401/06, C 07 D 401/12, C 07 C 103/76

(54) **Médicaments à base de dérivés de naphtalène- ou azanaphtalènecarboxamide, nouveaux dérivés de naphtalène- ou azanaphtalènecarboxamide et procédés pour leur préparation.**

(30) Priorité: **24.12.82 FR 8221758**

(43) Date de publication de la demande: **04.07.84 Bulletin 84/27**

(45) Mention de la délivrance du brevet: **22.07.87 Bulletin 87/30**

(84) Etats contractants désignés: **AT BE CH DE FR GB IT LI LU NL SE**

(56) Documents cité: **GB-A-10 352**
**US-A-3 799 929**

(73) Titulaire: **RHONE- POULENC SANTE, Les Miroirs 18 Avenue d'Alsace, F-92400 Courbevoie Cedex (FR)**

(72) Inventeur: **Dubroeucq, Marie- Christine, 13 Villa Malleville, F-95880 Enghien- Les- Bains (FR)**
Inventeur: **Le Fur, Gérard, 35 rue du Progrès, F-92350 Le Plessis- Robinson (DE)**
Inventeur: **Renault, Christian, 61 rue des Mallets, F-95150 Taverny (FR)**

(74) Mandataire: **Gaumont, Robert, RHONE- POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

EP 0 112 776 B1

**Description**

La présente invention a pour objet de nouveaux médicaments, particulièrement utiles pour le traitement des états d'anxiété et des troubles pulmonaires, rénaux, cardiovasculaires ou circulatoires, qui contiennent, en tant que substance active, un composé répondant à la formule:

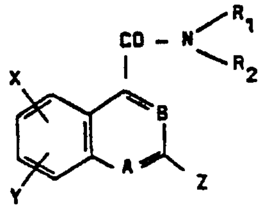

(I)

dans laquelle $R_1$ représente un groupe alkyle linéaire ou ramifié ayant 1 à 6 atomes de carbone, un groupe phényle, un groupe cycloalkyle ayant 3 à 6 atomes de carbone, un groupe phénylalkyle ou cycloalkylalkyle dont la partie alkyle comporte 1 à 3 atomes de carbone, ou un groupe

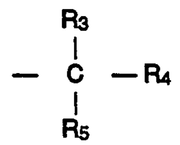

dans lequel $R_3$ et $R_4$ sont des atomes d'hydrogène ou des groupes alkyle et $R_5$ est un groupe alcényle ou alcinyle, la somme des atomes de carbone de $R_3$, $R_4$ et $R_5$ étant de 2 à 5, $R_2$ représente un groupe alkyle linéaire ou ramifié ayant 1 à 6 atomes de carbone, un groupe phényle, un groupe cycloalkyle ayant 3 à 6 atomes de carbone, un groupe phénylalkyle ou cycloalkylalkyle dont la partie alkyle comporte 1 à 3 atomes de carbone, un groupe,

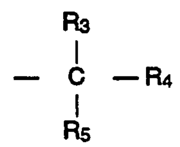

dans lequel $R_3$, $R_4$ et $R_5$ sont tels que définis ci-dessus, ou un groupe $-(CH_2)_n-$ 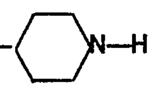

dans lequel n est 0, 1, 2 ou 3, $R_1$ et $R_2$ pouvant en outre former ensemble avec l'atome d'azote auquel ils sont rattachés un radical hétérocyclique à 5, 6 ou 7 chaînons pouvant contenir un autre hétéroatome choisi parmi l'azote et l'oxygène et pouvant porter un ou deux substituants choisis parmi les groupes alkyle ayant 1 à 3 atomes da carbone, le groupe hydroxy, le groupe oxo et les groupes hydroxyalkyle, diméthylaminoalkyle et diéthylaminoalkyle dont la partie alkyle comporte 1 à 3 atomes de carbone,

Z représente un groupe phényle, pyridyle, thiényle, thiazolyl-2, ou un groupe phényle substitué par un ou deux substituants pris parmi les atomes d'halogène (chlore, fluor, brome), les groupes alkyle, alkoxy et alkylthio ayant 1 à 3 atomes de carbone, le groupe trifluorométhyle et le groupe nitro,

X et Y sont identiques ou différents et représentent des atomes d'hydrogène ou d'halogène (fluor, chlore, brome), des groupes alkyle ou alkoxy ayant 1 à 3 atomes de carbone, des groupes nitro ou trifluorométhyle,

A représente un atome d'azote ou un groupe CH et B représente un atome d'azote ou un groupe CH, à l'exception du N,N-diéthyl phényl-2 quinoléine carboxamide-4, ou un mélange de composés stéréoisomères répondant à la formule (I), ou un sel d'un tel composé ou mélange de composés stéréoisomères avec un acide pharmaceutiquement acceptable.

Comme exemples de radical hétérocyclique que peuvent former $R_1$ et $R_2$ avec l'atome d'azote auquel ils sont liés on peut citer en particulier les radicaux pyrrolidinyle, pipéridino, morpholino, pipérazinyle, N-méthyl pipérazinyle, tétrahydro-1,2,3,6 pyridyle, hexahydro-2,3,4,5,6,7 azépinyle, oxo-4 pipéridino, pipéridino substitué par un ou deux groupes alkyle ayant 1 à 3 atomes de carbone ou par un groupe hydroxy en position 3 ou 4 ou encore par un groupe hydroxyalkyle, diméthylaminoalkyle ou diéthylaminoalkyle dont la partie alkyle a 1 à 3 atomes de carbone.

Dans la formule (I) ci-dessus X et Y sont de préférence des atomes d'hydrogène.

Lorsque le groupe 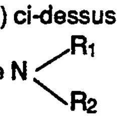

comporte un ou plusieurs atomes de carbone asymétriques, pour une signification donnée de X, Y, Z, A, B,

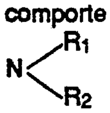

il y a plusieurs stéréoisomères correspondant à la formule plane (I).

Les composés de formule (I) pour lesquels A est un atome d'azote, B est un groupe CH, Z est un groupe phényle, X et Y sont des atomes d'hydrogène et $R_1$ et $R_2$ sont des groupes alkyle identiques ayant 1 à 4 atomes de carbone ou forment avec l'atome d'azote auxquels ils sont liés un groupe pipérazino sont des produits connus. (cf. brevet anglais 10 352; Il Farmaco, vol. 29, 1974, 507-516, article de G. PAGANI et Coll.; ROUSHDI et Coll., J. Pharm. Sci. U. Arab. Rep. 1961, 2, 109; WHITE et Coll., J. Org. Chem., 1942, 7, 497; SANNA, Chem. Zentr., 1941, 1, 1421). Les composés de formule (I) pour lesquels A et B sont des groupes CH, Z est un groupe phényle ou chloro-3 phényle, X est un atome d'hydrogène ou de chlore ou un groupe trifluorométhyle, Y est un atome d'hydrogène, et $R_1$ et $R_2$ sont des groupes méthyle sont également connus (cf. R.F. ABDULLA et Coll., J. Org. Chem.; 1980, 45, 1724-1725). Enfin le composé de formule (I) pour lequel A = N, B = CH, Z = méthyl-4 phényl, X = Y = H, $R_1 = R_2 = C_2H_5$ est connu (cf. JOHN et Coll., J. Pract. Chem., 1931, 131, 301). Parmi les composés de formule (I) connus, seul le N,N-diéthyl phényl-2 quinoléine-carboxamide-4 a été signalé comme ayant des propriétés pharmacologiques, plus précisément des propriétés uricosuriques et antipyrétiques (GHAZAL et Coll., Egypt. Pharm. Bull., 1960, 42, 465).

Les composés de formule (I) autres que ceux cités précédemment sont nouveaux et font partie en tant que tels de l'invention. Sont nouveaux en particulier les composés de formule (I) pour lesquels A et B sont des atomes d'azote et X, Y, Z, $R_1$ et $R_2$ ont les significations précitées, et ceux pour lesquels A est un groupe CH, B est un atome d'azote et X, Y, Z, $R_1$ et $R_2$ ont les significations précitées. Sont nouveaux également les composés de formule (I) pour lesquels A et B sont des groupes CH, $R_1$ est un groupe alkyle linéaire ou ramifié 2-6C, phényle, cycloalkyle 3-6C, phénylalkyle ou cycloalkylalkyle dont la partie alkyle a 1 à 3 atomes de carbone, ou

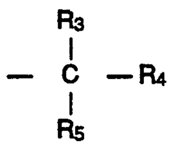

$R_3$, $R_4$ et $R_5$ ayant les significations précitées, $R_2$ est un groupe alkyle linéaire ou ramifié 1-6C, phényle, cycloalkyle 3-6C, phénylalkyle ou cycloalkylalkyle dont la partie alkyle a 1 à 3 atomes de carbone,

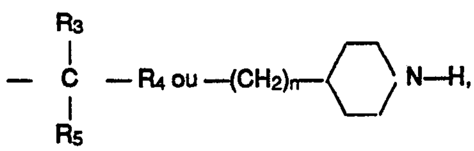

$R_3$, $R_4$, $R_5$ et n ayant les significations précitées, $R_1$ et $R_2$ pouvant en outre former ensemble avec l'atome d'azote auquel ils sont rattachés un radical hétérocyclique à 5, 6 ou 7 chaînons pouvant contenir un autre hétéroatome choisi parmi l'azote et l'oxygène et pouvant porter un ou deux substituants choisis parmi les groupes alkyle 1-3C, hydroxy, oxo, hydroxyalkyle, diméthylaminoalkyle et diéthylaminoalkyle dont la partie alkyle a 1 à 3 atomes de carbone, et Z, X et Y ont les significations précitées. Sont nouveaux aussi les composés de formule (I) pour lesquels A est un atome d'azote, B est un groupe CH, Z, X et Y ont les significations précitées, $R_1$ est un groupe phényle, cycloalkyle 3-6C, phénylalkyle ou cycloalkylalkyle dont la partie alkyle a 1 à 3 atomes de carbone, ou

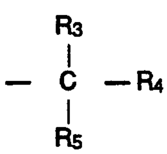

$R_3$, $R_4$ et $R_5$ ayant les significations précitées, $R_2$ est un groupe phényle, cycloalkyle 3-6C, phénylalkyle ou cycloalkylalkyle dont la partie alkyle a 1 à 3 atomes de carbone,

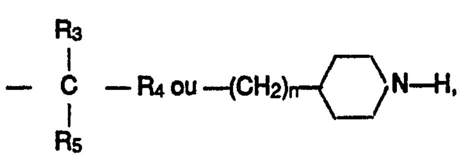

$R_3$, $R_4$, $R_5$ et n ayant les significations précitées, $R_1$ et $R_2$ pouvant en outre former ensemble avec l'atome d'azote auquel ils sont rattachés un radical pyrrolidinyle, pipéridino, morpholino, tétrahydro-1,2,3,6 pyridyle, hexahydro-2,3,4,5,6,7 azépinyle, oxo-4 pipéridino ou pipéridino substitué par un ou deux groupes alkyle 1-3C ou par un groupe hydroxy en position 3 ou 4 ou par un groupe hydroxyalkyle, diméthylaminoalkyle ou diéthylaminoalkyle dont la partie alkyle a 1 à 3 atomes de carbone, $R_1$ et $R_2$ peuvent aussi être des groupes alkyle différents 1-6C.

Les composés de formule générale (I) en particulier ceux qui sont nouveaux, peuvent être préparés par action d'une amine de formule 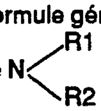 (II)

dans laquelle $R_1$ et $R_2$ ont les mêmes significations que dans la formule (I), sur un composé de formule:

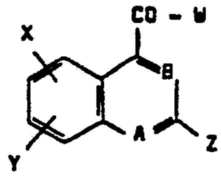

(III)

dans laquelle X, Y, Z, A et B ont les mêmes significations que dans la formule (I) et W représente un groupe alkoxy de bas poids moléculaire, un atome de chlore ou un groupe alcoxycarbonyloxy de bas poids moléculaire, suivant le schéma réactionnel suivant:

(1) (II) + (III) → (I) + WH

La réaction (1) est réalisés suivant des procédés, connus en soi, permettant de transformer un ester d'acide carboxylique, un chlorure d'acide carboxylique ou un anhydride mixte en carboxamide (cf. par exemple C.A. BUEHLER et D.C. PEARSON, Survey of Organic Synthesis, Wiley Interscience, 1970, page 804).

Lorsque W est un groupe alcoxy de bas poids moléculaire, on peut par exemple traiter l'ester de formule (III) par une quantité au moins équimoléculaire de l'amine de formule (II), en présence d'un agent de métallation tel que le butyllithium et au sein d'un solvant inerte tel que l'oxyde diéthylique ou le tétrahydrofuranne, à une température de -70°C à +30°C, ou chauffer l'ester de formule (III) au sein de l'amine de formule (II) en excès à une température de 100°C à 180°C, en l'absence d'agent de métallation.

Lorsque W est un atome de chlore, on peut par exemple traiter le chlorure d'acide de formule (III) par un excès de l'amine de formule (II) au sein d'un solvant inerte tel que le toluène, le chloroforme ou le chlorure de méthylène, à une température comprise entre 20°C et la température d'ébullition du solvant utilisé. L'excès de l'amine de formule (II) utilisé, qui joue le rôle de base neutralisant l'acide chlorhydrique formé dans la réaction, est d'au moins un équivalent, c'est-à-dire que la quantité totale d'amine de formule (II) employée est au moins deux équivalents. Dans le cas où au moins l'une des lettres A et B dans la formule (III) représente un atome d'azote, le chlorure d'acide de formule (III) peut être utilisé sous forme de chlorhydrate à condition d'employer au moins un équivalent supplémentaire de l'amine de formule (II) afin de faire passer le chlorure d'acide de formule (III) de la forme chlorhydrate à la forme base libre.

Lorsque W est un atome de chlore, on peut aussi faire réagir le chlorure d'acide de formule (III) avec l'amine de formule (II) en présence d'une amine tertiaire telle que la triéthylamine, au sein d'un solvant inerte tel que ceux cités précédemment et à une température comprise entre 20°C et la température d'ébullition du solvant. On peut aussi faire réagir le chlorure d'acide de formule (III) avec l'amine de formule (II) au sein de la pyridine, qui sert à la fois de base fixant l'acide formé et de solvant.

Lorsque W est un groupe alcoxycarbonyloxy de bas poids moléculaire, on peut par exemple traiter l'anhydride mixte de formule (III) par l'amine de formule (II) au sein d'un solvant inerte tel que le benzène, le toluène, le chloroforme ou le chlorure de méthylène, à une température de -5°C à +25°C.

Les composés de formule (III) peuvent être obtenus par action, sur un acide de formule:

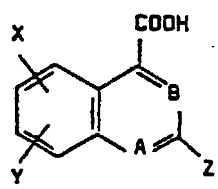

(IV)

dans laquelle X, Y, Z, A et B ont les mêmes significations que dans la formule (I), d'un alcool aliphatique saturé de bas poids moléculaire tel que le méthanol ou l'éthanol (cas où W est un groupe alcoxy de bas poids moléculaire), d'un agent de chloruration tel que le chlorure de thionyle (cas où W est un atome de chlore), ou d'un chloroformiate d'alkyle de bas poids moléculaire tel que le chloroformiate de méthyle ou le chloroformiate d'éthyle (cas où W est un groupe alcoxycarbonyloxy de bas poids moléculaire).

La réaction de l'acide de formule (IV) avec l'alcool aliphatique saturé de bas poids moléculaire peut être réalisée en chauffant à la température de reflux l'acide de formule (IV) au sein dudit alcool, en présence d'un acide minéral tel que l'acide sulfurique ou l'acide chlorhydrique.

La réaction de l'acide de formule (IV) avec l'agent de chloruration peut être réalisée en l'absence de solvant ou au sein d'un solvant inerte tel que le chloroforme, de préférence à la température de reflux du milieu.

La réaction de l'acide de formule (IV) avec le chloroformiate d'alkyle de bas poids moléculaire peut être

réalisée au sein d'un solvant inerte tel que le chloroforme ou le chlorure de méthylène, à une température de -5°C à +25°C, en présence d'une amine tertiaire telle que la triéthylamine. On peut ensuite faire réagir in situ, c'est-à-dire sans isolement préalable, l'anhydride mixte ainsi formé avec l'amine de formule (II).

Les composés de formule (I) pour lesquels $R_2$ est un groupe $—(CH_2)_n—$ 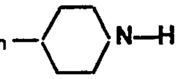

peuvent aussi être préparés par action d'une amine de formule:

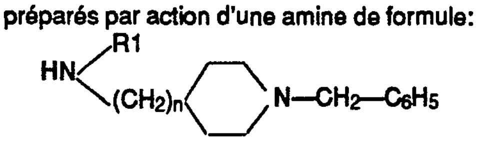 (V)

dans laquelle n et $R_1$ ont les mêmes significations que dans la formule (I), sur un composé de formule (III), puis débenzylation de l'amide de formule (VI) ainsi obtenue, suivant le schéma réactionnel suivant:

(2) (III) + (V) → 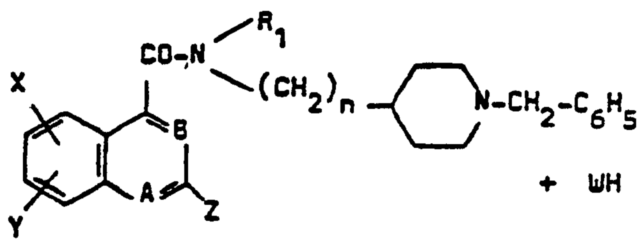 + WH

(3) (VI) (I) —débenzylation→ avec $R_2$ = $—(CH_2)_n—$ 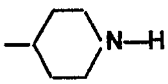

La réaction (2) est réalisée dans les mêmes conditions que la réaction (1). La réaction (3) (débenzylation) est effectuée suivant des procédés, connus en soi, permettant de débenzyler les N-benzylamines tertiaires. On peut par exemple faire réagir sur le composé de formule (VI) soit l'hydrogène en présence d'un catalyseur tel que le palladium (cf. P.N. RYLANDER, Catalytic Hydrogenation over Platinium Metals, Academic Press, 1967, p.449), soit le chloroformiate de trichloro-2,2,2 éthyle (cf. M.C. REINECKE et Coll., J. Org. Chem., 1973, 38, 3281).

Un grand nombre d'acides carboxyliques répondant à la formule (IV) sont connus. Ceux qui ne le sont pas peuvent être aisément préparés en appliquant aux produits de départ appropriés les méthodes précédemment utilisées pour la synthèse des acides de formule (IV) connus (cf. par exemple les documents mentionnés plus loin dans les exemples 2, 6, 7, 9, 10, 12 à 14, 20, 24, 35 et 54).

Les mélanges réactionnels obtenus par les divers procédés décrits précédemment sont traités suivant des méthodes classiques, physiques (évaporation, extraction à l'aide d'un solvant, distillation, cristallisation, chromatographie,) ou chimiques (formation de sel et régénération de la base) afin d'isoler les composés de formule (I) à l'état pur.

Les composés de formule (I) sous forme de base libre peuvent éventuellement être transformés en sels d'addition avec un acide minéral ou organique par action d'un tel acide au sein d'un solvant approprié.

Les divers effets cliniques (anxiolytique, anticonvulsivant, hypnotique, myorelaxant) des benzodiazépines ont été attribués à la présence, dans le système nerveux central des mammifères, de sites de liaison saturables, à haute affinité et stéréospécifiques (C. BRAESTRUP et Coll., Nature, 1977, 269, 702; J.F. TALLMAN et Coll., Science, 1980, 207, 274).

Certaines benzodiazépines se liant aussi à des membranes de tissus périphériques comme le rein avec également une forte affinité (C. BRAESTRUP et Coll., Proc. Natl. Acad. Sci. USA, 1977, 74, 3805). Les récepteurs de benzodiazépines présents dans ces tissus diffèrent de ceux marqués par le [$^3$H] diazépam ou le [$^3$H] flunitrazépam dans le cerveau. Par exemple, le clonazépam, qui a une très forte affinité pour les sites de liaison du [$^3$H] diazépam du cerveau, est pratiquement inactif à l'égard des sites de liasion du [$^3$H] diazépam dans le rein. A l'inverse un dérivé chloré du diazépam, le Ro-5-4864, est très actif au niveau périphérique mais inactif au niveau central. Ainsi il est possible de distinguer au moins 2 types de récepteurs de benzodiazépines, l'un de type "cérébral", dont le critère pharmacologique sera un classement par affinité décroissante dans l'ordre clonazépam > diazépam > Ro-5-4864, et l'autre de type "périphérique", dont le critère pharmacologique sera un classement par affinité décroissant dans l'ordre Ro-5-4864 > diazépam > clonazépam.

Ces récepteurs de type "périphérique" sont présents dans de nombreux organes: le coeur, le rein, le poumon, les plaquettes sanguines et égalemant le cerveau (où sont donc présents les 2 types de récepteurs) (cf, L.P. DAVIES et Coll., Eur. J. Pharmacol., 1981, 73, 209; J.K.T. WANG et Coll., Life Sciences, 1980, 27, 1881; J.W. REGAN et Coll., Life Sciences, 1981, 28, 991; H. SCHOEMAKER, Eur. J. Pharmacol., 1981, 71, 173).

Les composés de formule (I), bien qu'ayant une structure différente de celle des benzodiazépines, ont la propriété de se lier aux récepteurs des benzodiazépines. Selon leur structure ils se lient préférentiellement aux récepteurs cérébraux ou aux récepteurs périphériques. Par exemple, les composés de formule (I) pour lesquels X

et Y sont des atomes d'hydrogène, A est un atome d'azote, B est un atome d'azote ou un groupe CH, Z est le groupe phényle et $N\begin{smallmatrix} R_1 \\ R_2 \end{smallmatrix}$ est un groupe diéthylamino, pipéridino ou pyrrolidino agissent préférentiellement sur les récepteurs cérébraux, alors que le N,N-diéthyl phényl-3 naphtalènecarboxamide-1 et les composée de formule (I) pour lesquels X et Y sont des atomes d'hydrogène, Z est un groupe phényle ou chloro-2 (ou -3) phényle, et au moins l'un des substituants $R_1$ et $R_2$ est un groupe alkyle ayant 3 à 6 atomes de carbone ramifié en position α par rapport à l'atome d'azote agissent préférentiellement sur les récepteurs périphériques.

Les exemples suivants illustrent la préparation des composés de formule (I), substances actives des médicaments selon l'invention.

**Exemple 1:** (signale pour référence aux exemples suivants): N,N-diéthyl phényl-2 quinoléinecarboxamide-4

On porte à l'ébullition 30 g d'acide phényl-2 quinoléinecarboxylique-4 dans 100 ml de chlorure de thionyle. Après une heure de chauffage au reflux, on évapore le chlorure de thionyle, et reprend le résidu dans 100 ml de toluène, que l'on évapore de nouveau.

On ajoute alors au résidu obtenu 100 ml de toluène puis, sous agitation, 70 ml de diéthylamine. On chauffe au reflux pendant une heure, puis on verse le mélange réactionnel dans 500 ml d'eau. On décante la phase organique et extrait la phase aqueuse par 3 fois 150 ml d'acétate d'éthyle. Les phases organiques sont rassemblées, séchées sur sulfate de magnésium et évaporées sous pression réduite. Après cristallisation du résidu obtenu dans l'oxyde diéthylique et recristallisation dans l'alcool isopropylique, on obtient 18 g de N,N-diéthyl phényl-2 quinoléinecarboxamide-4 fondant à 110°C.

**Exemple 2:** N,N-diéthyl (pyridyl-2)-2 quinoléinecarboxamide-4

On procède comme à l'exemple 1, en partant de 15 g d'acide (pyridyl-2)-2 quinoléinecarboxylique-4, de 45 ml de chlorure de thionyle et de 6 ml de diéthylamine.

Après recristallisation du résidu dans l'éther isopropylique, on obtient 14,5 g de N,N-diéthyl (pyridyl-2)-2 quinoléinecarboxamide-4 fondant à 100°C.

L'acide (pyridyl-2)-2 quinoléinacarboxylique-4 peut être préparé selon le procédé décrit par RISALITI, Ric., Scient., 28 (1958), 561, 563.

**Exemple 3:** [(Pyridyl-2)-2 quinolyl-4] carbonyl-1 pipéridine

On chauffe au reflux pendant une heure 3 g d'acide (pyridyl-2)-2 quinoléinecarboxylique-4 et 10 ml de chlorure de thionyle. On évapore le chlorure de thionyle, reprend le résidu par 100 ml de toluène et évapore à nouveau. On ajoute alors au résidu obtenu 20 ml de toluène puis, goutte-à-goutte, sous agitation, 3,5 ml de pipéridine. On agite deux heures à température ambiante. Le mélange réactionnel est ensuite coulé dans 50 ml d'eau. La phase organique est décantée et la phase aqueuse est extraite par trois fois 100 ml d'acétate d'éthyle. Les phases organiques sont rassemblées, lavées par trois fois 30 ml d'eau, séchées sur sulfate de magnésium et évaporées sous pression réduite.

Après recristallisation du résidu obtenu dans l'acétate d'éthyle, on obtient 2,9 g de [(pyridyl-2)-2 quinolyl-4] carbonyl-1 pipéridine fondant à 158°C.

**Exemple 4:** (Phényl-2 quinolyl-4) carbonyl-1 pipéridine

On procède comme à l'exemple 3, en partant de 8 g d'acide phényl-2 quinoléinecarboxylique-4, de 24 ml de chlorure de thionyle et de 8,16 g de pipéridine.

Après recristallisation du résidu dans l'éther isopropylique, on isole 8,7 g de (phényl-2 quinolyl-4) carbonyl-1 pipéridine fondant à 104°C.

**Exemple 5:** (Phényl-2 quinolyl-4) carbonyl-4 Morpholine

On procède comme à l'example 3, en partant de 8 g d'acide phényl-2 quinoléinecarboxylique-4, de 24 ml de chlorure de thionyle et de 8,3 g de morpholine.

Après recristallisation dans l'éthanol, on obtient 8 g de (phényl-2 quinolyl-4) carbonyl-4 morpholine fondant à 124°C.

**Exemple 6:** N,N-diéthyl (chloro-4 phényl)-2 quinoléinecarboxamide-4

On procède comme à l'exemple 3, en partant de 5 g d'acide (chloro-4 phényl)-2 quinoléinecarboxylique-4, de 15 ml de chlorure de thionyle et de 18 ml de diéthylamine.

Après recristallisation du résidu dans l'éther isopropylique, on isole 4,2 g de N,N-diéthyl (chloro-4 phényl)-2 quinoléinecarboxamide-4 fondant à 105°C.

L'acide (chloro-4 phényl)-2 quinoléinecarboxylique-4 peut être préparé sslon le procédé décrit par R.F. BROWN et al. J. Amer. Chem. Soc. 68, 2705 (1946).

**Exemple 7:** N,N-diéthyl (méthoxy-4 phényl)-2 quinoléinecarboxamide-4

On procède comme à l'exemple 3, en partant de 5 g d'acide (méthoxy-4 phényl)-2 quinoléinecarboxylique-4, de 15 ml de chlorure de thionyle et de 18,4 ml de diéthylamine.

Après recristallisation du résidu dans l'éther isopropylique, on isole 3,8 g de N,N-diéthyl (méthoxy-4 phényl)-2 quinoléinecarboxamide-4 fondant à 128°C.

L'acide (méthoxy-4 phényl)-2 quinoléinecarboxylique-4 peut être préparé selon le procédé décrit par CIUSA et LUZZATTO, Gazz. Chim. Ital., 44, 64 (1914).

**Exemple 8:** N,N-diéthyl (chloro-2 phényl)-2 quinoléinecarboxamide-4

On procède comme à l'exemple 3, en partant de 5 g d'acide (chloro-2 phényl)-2 quinoléinecarboxylique-4, de 15 ml de chlorure de thionyle et de 12,7 ml de diéthylamine.

Après recristallisation du résidu dans l'éther isopropylique, on isole 3,2 g de N,N-diéthyl (chloro-2 phényl)-2 quinoléinecarboxamide-4 fondant à 130°C.

L'acide (chloro-2 phényl)-2 quinoléinecarboxylique-4 peut être préparé selon le procédé décrit par R.F. BROWN et al., J. Amer. Chem. Soc. 68, 2705 (1946).

**Exemple 9:** N,N-diéthyl (trifluorométhyl-3 phényl)-2 quinoléinecarboxamide-4

On procède comme à l'exemple 3, en partant de 6 g d'acide (trifluorométhyl-3 phényl)-2 quinoléinecarboxylique-4, de 18 ml de chlorure de thionyle et de 18,5 ml de diéthylamine.

Après recristallisation du résidu dans l'éther isopropylique, on isole 5,2 g de N,N-diéthyl (trifluorométhyl-3-phényl)-2 quinoléinecarboxamide-4 fondant à 100°C.

L'acide (trifluorométhyl-3 phényl)-2 quinoléinecarboxylique-4 peut être préparé selon le procédé décrit par SHARGIER et LALEZARI, J. Chem. Eng. Data, 8, 276 (1963).

**Exemple 10:** N,N-diéthyl (fluoro-4 phényl)-2 quinoléinecarboxamide-4

On procède comme à l'exemple 3, en partant de 5 g d'acide (fluoro-4 phényl)-2 quinoléinecarboxylique-4, de 15 ml de chlorure de thionyle et de 19,2 ml de diéthylamine.

Après chromatographie du résidu sur du gel de silice avec un éluant constitué par un mélange hexane-acétate d'éthyle (70 : 30) et recristallisation du produit ainsi séparé dans l'éther isopropylique, on isole 0,86 g de N,N-diéthyl (fluoro-4 phényl)-2 quinoléinecarboxamide-4 fondant à 114°C.

L'acide (fluoro-4 phényl)-2 quinoléinecarboxylique-4 peut être préparé selon le procédé décrit par Bu Hoï et al., Rec. Trav. Chim., 68, 781 (1949).

**Exemple 11:** (phényl-2 quinolyl-4) carbonyl-1 pyrrolidine

On procède comme à l'exemple 3, en partant de 10 g d'acide phényl-2 quinoléinecarboxylique-4, de 30 ml de chlorure de thionyle et de 10,3 ml de pyrrolidine. On isole ainsi, après recristallisation du résidu dans l'isopropanol, 5,6 g de (phényl-2 quinolyl-4) carbonyl-1 pyrrolidine fondant à 128°C.

**Exemple 12:** N,N-diéthyl diméthoxy-6,7 phényl-2 quinoléinecarboxamide-4

On opère comme à l'exemple 3, en partant de 2,5 g d'acide diméthoxy-6,7 phényl-2 quinoléinecarboxylique-4, de 8 ml de chlorure de thionyle et de 8,2 ml de diéthylamine.

On obtient ainsi 2,9 g de N,N-diéthyl diméthoxy-6,7 phényl-2 quinoléinecarboxamide-4 sous forme d'une huile, que l'on transforme, au sein de l'acétone, en son chlorhydrate par addition d'une solution d'acide chlorhydrique dans l'éther. Après recristallisation dans l'acétone, celui-ci présente un point de fusion égal à 140°C.

L'acide diméthoxy-6,7 phényl-2 quinoléinecarboxylique-4 peut être préparé selon le procédé décrit par BORSCHE ET BARTENHEIMER, Ann., 548, 50 (1941).

**Exemple 13:** N,N-diéthyl méthyl-6 phényl-2 quinoléinecarboxamide-4

On opère comme à l'exemple 3, en partant de 3,2 g d'acide méthyl-6 phényl-2 quinoléinecarboxylique-4, de 15 ml de chlorure de thionyle et de 12,5 ml de diéthylamine.

Après recristallisation du résidu dans l'éther isopropylique, on obtient 3,1 g de N,N-diéthyl méthyl-6 phényl-2 quinoléinecarboxamide-4 fondant à 120°C.

L'acide méthyl-6 phényl-2 quinoléinecarboxylique-4 peut être préparé selon le procédé décrit par BUCHMANN et HOWTON, J. Amer. Chem. Soc., 68, 2718 (1946).

**Exemple 14**: N,N-diéthyl nitro-8 phényl-2 quinoléinecarboxamide-4

On opère comme à l'exemple 3, en partant de 32 g d'acide nitro-8 phényl-2 quinoléinecarboxylique-4, de 9 ml de chlorure de thionyle et de 11,5 ml de diéthylamine.

Après recristallisation du résidu dans un mélange cyclohexane/acétate d'éthyle (1 : 1), on isole 2,45 g de N,N-diéthyl nitro-8 phényl-2 quinoléinecarboxamide-4 fondant à 138°C.

L'acide nitro-8 phényl-2 quinoléinecarboxylique-4 peut être préparé selon le procédé décrit par BUCHMANN et Al., J. Amer. Chem. Soc., 69, 380 (1947).

**Exemple 15**: N-méthyl N-(méthyl-1 propyl) (chloro-2 phényl)-2 quinoléinecarboxamide-4.

On opère comme à l'exemple 3, en partant de 5,7 g d'acide (chloro-2 phényl)-2 quinoléinecarboxylique-4, de 15 ml de chlorure de thionyle et de 15 ml de N-méthyl butanamine-2.

On obtient, après recristallisation du résidu dans l'éther isopropylique, 5 g de N-méthyl N-(méthyl-1 propyl) (chloro-2 phényl)-2 quinoléinecarboxamide-4 fondant à 118°C.

**Exemple 16**: N,N-di(méthyl-1 propyl) (chloro-2 phényl)-2 quinoléinecarboxamide-4

On opère comme à l'exemple 3, en partant de 2,83 g d'acide (chloro-2 phényl)-2 quinoléinecarboxylique-4, de 10 ml de chlorure de thionyle et de 5,16 g de N(méthyl-1 propyl) butanamine-2.

On obtient, après une première chromatographie du résidu sur du gel de silice avec un mélange cyclohexane-acétate d'éthyle (1 : 1) comme éluant, puis après une seconde chromatographie sous pression sur du gel de silice avec un mélange cyclohexane/acétate d'éthyle (7 : 3) comme éluant, 1,8 g de N,N-di (méthyl-1 propyl) (chloro-2 phényl)-2 quinoléinecarboxamide-4 fondant à 120°C.

**Exemple 17**: N-éthyl N-(méthyl-1 propyl) (chloro-2 phényl)-2 quinoléinecarboxamide-4

On opère comme à l'exemple 3, en partant de 2,8 g d'acide (chloro-2 phényl)-2 quinoléinecarboxylique-4, de 10 ml de chlorure de thionyle et de 4 g de N-éthyl butanamine-2.

On obtient, après recristallisation du résidu dans l'éther isopropylique, 0,9 g de N-éthyl N-(méthyl-1 propyl) (chloro-2 phényl)-2 quinoléinecarboxamide-4 fondant à 95°C.

**Exemple 18**: [(chloro-2 phényl)-2 quinolyl-4] carbonyl-1 pipéridine

On procède comme à l'exemple 3, en partant de 2,8 g d'acide (chloro-2 phényl)-2 quinoléinecarboxylique-4, de 10 ml de chlorure de thionyle et de 3,4 g de pipéridine.

Après recristallisation du résidu dans l'acétate d'éthyle, on isole 2,1 g de [(chloro-2 phényl)-2 quinolyl-4] carbonyl-1 pipéridine fondant à 129°C.

**Exemple 19**: N,N-diéthyl (thiazolyl-2)-2 quinoléinecarboxamide-4

On opère comme à l'exemple 3, en partant de 0,8 g d'acide (thiazolyl-2)-2 quinoléinecarboxylique-4, de 20 ml de chlorure de thionyle et de 10 ml de diéthylamine.

Après chromatographie du résidu sur du gel de silice en utilisant comme éluant un mélange chloroforme/acétate d'éthyle (9 : 1), on isole 0,3 g de N,N-diéthyl (thiazolyl-2)-2 quinoléinecarboxamide-4 fondant à 97°C.

L'acide (thiazolyl-2)-2 quinoléinecarboxylique-4 est obtenu par action de l'isatine (1,4 . $10^{-2}$ mole) sur l'acétyl-2 thiazoles (1,3 . $10^{-2}$ mole) dans un milieu constitué par 30 ml d'une solution aqueuse d'hydroxyde de potassium 6N et 10 ml d'éthanol, à la températurs de reflux. Il présente un point de fusion de 250°C.

L'acétyl-2 thiazole peut être obtenu selon le procédé décrit par ADAMSON et al., J. Chem. Soc., (1969), 2270-3.

**Exemple 20**: N,N-diéthyl phényl-3 naphtalènecarboxamide-1

On opère comme à l'exemple 3, en utilisant 5 g d'acide phényl-3 naphtalènecarboxylique-1 à la place de 3 g d'acide (pyridyl-2)-2 quinoléinecarboxylique-4, 20 ml de chlorure de thionyle à la place de 10 ml de chlorure de thionyle, et 4,5 ml de diéthylamine dans 25 ml de pyridine à la place de 3,5 ml de pipéridine dans 20 ml de toluène. Après recristallisation dans l'hexane, on obtient 3,4 g de N,N-diéthyl phényl-3 naphtalènecarboxamide-1, qui fond à 65°C.

L'acide phényl-3 naphtalènecarboxylique-1 peut être préparé selon le procédé décrit par F.G. BADDAR et Coll. J. Chem. Soc., 1959, 1009.

**Exemple 21**: N-méthyl N-(méthyl-1 propyl) phényl-3 naphtalènecarboxamide-1

On opère comme à l'exemple 20, en partant de 4,3 g d'acide phényl-3 naphtalènecarboxylique-1, 20 ml de

chlorure de thionyle, et 1,5 g de N-méthyl butanamine-2 dans 20 ml de pyridine. Après recristallisation dans l'éther de pétrole, on obtient 2,8 g de N-méthyl N-(méthyl-1 propyl) phényl-3 naphtalènecarboxamide-1 fondant à 125°C.

**Exemple 22:** N,N-diéthyl (chloro-2 phényl)-2 quinazoline carboxamide-4

On opère comme à l'exemple 3, en partant de 2,16 g d'acide (chloro-2 phényl)-2 quinazolinecarboxylique-4 et 10 ml de chlorure de thionyle, puis de 10 ml de diéthylamine dans 20 ml de toluène. Après recristallisation dans l'éther isopropylique, on obtient 2,2 g de N,N-diéthyl (chloro-2 phényl)-2 quinazolinecarboxamide-4 fondant à 124°C.

L'acide (chloro-2 phényl)-2 quinazolinecarboxylique-4 peut être préparé comme suit: on chauffe à 90°C, pendant 1 heure et 30 minutes, un mélange de 15 g de N-phényl chloro-2 benzamide et de 11 ml de chlorure de thionyle. On élimine l'excès de chlorure de thionyle par distillation sous pression réduite et on ajoute 7 g de cyanoformiate d'éthyle et 18,2 g de tétrachlorure d'étain. On porte à 140°C pendant 10 minutes, refroidit, verse le milieu réactionnel sur un mélange de chlorure de méthylène et d'eau, lave la phase organique à l'eau, la sèche sur du sulfate de magnésium et l'évapore à sec sous pression réduite. Par chromatographie du résidu sur du gel de silice avec un mélange cyclohexane/acétate d'éthyle (9 : 1) comme éluant, on isole 7 g d'un produit que l'on traite à l'ébullition par 10 ml d'hydroxyde de sodium 5N et 30 ml d'éthanol. On élimine l'éthanol par distillation sous pression réduite, reprend le résidu à l'eau et à l'éther. Par acidification de la phase aqueuse, on obtient 2,3 g d'acide (chloro-2 phényl)-2 quinazolinecarboxylique-4 fondant à 171°C.

**Exemple 23:** (phényl-2 quinolyl-4) carbonyl-1 pipérazine

On chauffe au reflux, pendant quatre heures, 30 g d'acide phényl-2 quinoléinecarboxylique-4 dans 90 ml de chlorure de thionyle. On évapore le chlorure de thionyle, reprend le résidu par 100 ml d'oxyde diéthylique et évapore à nouveau. Le résidu est ajouté par portions à une solution agitée de 51,6 g de pipérazine dans 250 ml de chlorure de méthylène. On agite une nuit à la température ambiante. La solution est alors reprise par 500 ml de chlorure de méthylène et 400 ml d'eau. La phase organique est séparée et la phase aqueuse est lavée par 3 fois 100 ml de chlorure de méthylène. Les phases organiques sont rassemblées, lavées par 150 ml d'eau, séchées sur sulfate de magnésium et évaporées sous pression réduite.

Le résidu est repris par 200 ml d'une solution aqueuse 0,1N d'acide acétique. L'insoluble est filtré, lavé par 3 fois 20 ml de la solution aqueuse 0,1N d'acide acétique. Le filtrat et les solutions de lavage sont rassemblés et alcalinisés par addition d'une solution concentrée d'hydroxyde de potassium, et on extrait par 3 fois 150 ml de chlorure de méthylène. La phase organique est lavée par 3 fois 60 ml d'eau, séchée sur sulfate de magnésium et évaporée sous pression réduite. Le résidu est chromatographié sur du gel de silice en utilisant comme éluant un mélange toluène/diéthylamine 9 : 1.

La base brute ainsi obtenue est mise en solution dans l'éthanol, puis transformée en son dichlorhydrate par addition d'une solution d'acide chlorhydrique dans l'éther. Après recristallisation dans l'éthanol, on obtient 2,4 g de dichlorhydrate de (phényl-2 quinolyl-4) carbonyl-1 pipérazine, fondant au-dessus de 250°C.

**Exemple 24:** N,N-diéthyl (méthyl-4 phényl)-2 quinoléinecarboxamide-4

A 5,5 ml de diéthylamine dans 50 ml de tétrahydrofuranne anhydre, on ajoute, à température ambiante et sous atmosphère d'azote, 22,5 ml d'une solution 1,6 M de butyllithium dans l'hexane. Après 15 minutes d'agitation, on refroidit à 0°C, puis on introduit lentement 5,25 g de (méthyl-4 phényl)-2 quinoléinecarboxylate-4 d'éthyle. On agite 2 heures à température ambiante. On ajoute alors lentement, à température ambiante et sous agitation, de l'acide acétique jusqu'à décoloration et obtention d'une solution jaune. On ajoute ensuite 100 ml d'eau, évapore le tétrahydrofuranne sous pression réduite, et extrait par 3 fois 100 ml d'acétate d'éthyle. La phase organique est lavée à l'eau, séchée sur sulfate de magnésium et évaporée sous pression réduite. Le résidu est chromatographié sur du gel de silice avec un mélange cyclohexane/acétate d'éthyle (80 : 20) comme éluant. Après recristallisation du produit brut ainsi isolé dans l'éther isopropylique, on obtient 1,1 g de N,N-diéthyl (méthyl-4 phényl)-2 quinoléinecarboxamide-4 fondant à 145°C.

Le (méthyl-4 phényl)-2 quinoléinecarboxylate-4 d'éthyle peut être préparé par action de l'éthanol sur l'acide (méthyl-4 phényl)-2 quinoléinecarboxylique-4 en présence d'acide sulfurique concentré. Il présente un point de fusion de 52°C.

L'acide (méthyl-4 phényl)-2 quinoléinecaboxylique-4 peut être préparé selon le procédé décrit par C. PREVOST et al., Compt. Rend. Acad. Sci., 258, 954 (1964).

**Exemple 25:** N,N-diéthyl phényl-2 quinazolinecarboxamide-4

On opère comme à l'exemple 24 en partant de 2,9 g de phényl-2 quinazolinecarboxylate-4 d'éthyle, de 3 ml de diéthylamine, de 10 ml de solution 1,6 M de butyllithium dans l'hexane, et de 10 ml de tétrahydrofuranne. Après recristallisation dans l'éthanol, on obtient 2,2 g de N,N-diéthyl phényl-2 quinazolinecarboxamide-4 fondant à 127°C.

Le phényl-2 quinazolinecarboxylate-4 d'éthyle peut être préparé selon le procédé de H. MEERWEIN et Coll.

Chem. Ber., 1956, 89, 224, en évitant l'hydrolyse en acide. Il a un point de fusion de 55-56°C.

**Exemple 26:** [(phényl-2 quinazolinyl-4) carbonyl]-1 pyrrolidine

On opère comme à l'exemple 24, en partant de 3 g de phényl-2 quinazolinecarboxylate-4 d'éthyle, de 3 ml de pyrrolidine, de 15 ml de solution 1,6 M de butyllithium dans l'hexane, et de 25 ml de tétrahydrofuranne. Après recristallisation dans l'éthanol, on obtient 2,1 g de [(phényl-2 quinazolinyl-4) carbonyl]-1 pyrrolidine fondant à 153°C.

**Exemple 27:** [(phényl-2 quinazolinyl-4) carbonyl]-1 pipéridine

On opère comme à l'exemple 24, à partir de 3,3 g de phényl-2 quinazolinecarboxylate-4 d'éthyle, de 2,5 ml de pipéridine, de 15 ml de solution 1,6 M de butyllithium dans l'hexane, et de 20 ml de tétrahydrofuranne. Après recristallisation dans l'éthanol, on obtient 2,6 g de [(phényl-2 quinazolinyl-4) carbonyl]-1 pipéridine fondant à 160°C.

**Exemple 28:** [(phényl-2 quinazolinyl-4) carbonyl]-4 morpholine

On opère comme à l'exemple 24, à partir de 3,1 g de phényl-2 quinazolinecarboxyate-4 d'éthyle, de 2 ml de morpholine, de 15 ml de solution 1,6M de butyllithium dans l'hexane, et de 20 ml de tétrahydrofuranne. Après recristallisation dans l'éthanol, on obtient 3 g de [(phényl-2 quinazolinyl-4) carbonyl]-4 morpholine fondant à 148°C.

**Exemple 29:** [(phényl-2 quinazolinyl-4) carbonyl]-1 pipéridinol-4

On opère comme à l'exemple 24, à partir de 2 g de phényl-2 quinazolinecarboxylate-4 d'éthyle, de 1,4 g de pipéridinol-4, de 18 ml de solution 1,6M de butyllithium dans l'hexane, et de 30 ml de tétrahydrofuranne. Après recristallisation dans un mélange de méthanol et de chlorure de méthylène, on obtient 2,1 g de [(phényl-2 quinazolinyl-4) carbonyl]-1 pipéridinol-4 fondant à 209°C.

**Exemple 30:** Hexahydro-2,3,4,5,6,7 [(phényl-2 quinazolinyl-4) carbonyl]-1 azépine

On opère comme dans l'exemple 24, à partir de 3 g de phényl-2 quinazolinecarboxylate-4 d'éthyle, de 2,8 ml d'hexahydro-2,3,4,5,6,7 azépine, de 15 ml de solution 1,6M de butyllithium dans l'hexane, et de 20 ml de tétrahydrofuranne. Après recristallisation dans l'éthanol, on obtient 2 g d'hexahydro-2,3,4,5,6,7 [(phényl-2 quinazolinyl-4) carbonyl]-1 azépine fondant à 140°C.

**Exemple 31:** méthyl-4 [(phényl-2 quinazolinyl-4)carbonyl]-1 pipéridine

On opère comme à l'exemple 24, à partir de 3 g de phényl-2 quinazolinecarboxylate-4 d'éthyle, de 2,95 ml de méthyl-4 pipéridine, de 15 ml de solution 1,6M de butyllithium dans l'hexane, et de 20 ml de tetrahydrofuranne. Après recristallisation dans l'éthanol, on obtient 1 g de méthyl-4 [(phényl-2 quinazolinyl-4) carbonyl]-1 pipéridine fondant à 138°C.

**Exemple 32:** Méthyl-3 [(phényl-2 quinazolinyl-4) carbonyl]-1 pipéridine

On opère comme à l'exemple 24, à partir de 3 g de phényl-2 quinazolinecarboxylate-4 d'éthyle, de 3 ml de méthyl-3 pipéridine, de 15 ml de solution 1,6M de butyllithium dans l'hexane, et de 20 ml de tétrahydrofuranne. Après recristallisation dans l'éther isopropylique, on obtient 1 g de méthyl-3 [(phényl-2 quinazolinyl-4) carbonyl]-1 pipéridine fondant à 116°C.

**Exemple 33:** Méthyl-2 [(phényl-2 quinazolinyl-4) carbonyl]-1 pipéridine

On opère comme à l'exemple 24, à partir de 3 g de phényl-2 quinazolinecarboxylate-4 d'éthyle, de 3 ml de méthyl-2 pipéridine, de 15 ml de solution 1,6M de butyllithium dans l'hexane, et de 20 ml de tétrahydrofuranne. Après recristallisation dans l'éthanol, on obtient 1,7 g de méthyl-2 [(phényl-2 quinazolinyl-4) carbonyl]-1 pipéridine fondant à 150°C.

**Exemple 34:** [(phényl-2 quinazolinyl-4) carbonyl]-1 pipéridine-3 méthanol

On opère comme à l'exemple 24, à partir de 3 g de phényl-2 quinazolinecarboxylate-4 d'éthyle, de 2,9 g de pipéridine-3 méthanol, de 31 ml de solution 1,6M de butyllithium dans l'hexane, et de 20 ml de tétrahydrofuranne. Après recristallisation dans l'acétate d'éthyle, on obtient 1 g de [(phényl-2 quinazolinyl-4) carbonyl]-1 pipéridine-3 méthanol fondant à 142°C.

**Exemple 35:** N,N-diéthyl chloro-6 (chloro-4 phényl)-2 quinoléinecarboxamide-4

A 3 ml de diéthylamine dans 15 ml de tétrahydrofuranne anhydre, on ajoute, à température ambiante et sous atmosphère d'azote, 12,5 ml d'une solution 1,6M de butyllithium dans l'hexane. Après une heure d'agitation, on refroidit à -65°C, puis on introduit lentement 3,4 g de chloro-6 (chloro-4 phényl)-2 quinoléine-carboxylate-4 d'éthyle dans 15 ml de tétrahydrofuranne. On agite 2 h 30 mn. à -65°C, puis on introduit 4 ml d'acide acétique glacial, laisse la température du milieu revenir à -10°C, ajoute 50 ml d'eau et laisse enfin revenir à température ambiante. On évapore le tétrahydrofuranne, extrait l'insoluble par 3 fois 50 ml d'acétate d'éthyle. La phase organique est lavée à l'eau, séchée sur sulfate de magnésium et évaporée sous pression réduite.

Le résidu obtenu est chromatographié sur du gel de silice avec un mélange cyclohexane/chloroforme (50 : 50) comme éluant. Après recristallisation dans l'acétate d'éthyle du produit brut ainsi isolé, on obtient 1,1 g de N,N-diéthyl chloro-6 (chloro-4 phényl)-2 quinoléinecarboxamide-4 fondant à 173°C.

Le chloro-6 (chloro-4 phényl)-2 quinoléinecarboxylate-4 d'éthyle peut être préparé par action de l'éthanol sur l'acide chloro-6 (chloro-4 phényl)-2 quinoléinecarboxylique-4, en présence d'acide sulfurique concentré. Il présente un point de fusion de 130°C.

L'acide chloro-6 (chloro-4 phényl)-2 quinoléinecarboxylique-4 peut être synthétisé selon le procédé décrit par LUTZ et al., J. Amer. Chem. Soc. 68 (1946) 1813-14.

**Exemple 36:** N,N-diéthyl (chloro-3 phényl)-2 quinoléinecarboxamide-4

On procède comme à l'exemple 35, en partant de 4,3 ml de diéthylamine, 20 ml d'une solution 1,6M de butyllithium dans l'hexane, et 3,2 g de (chloro-3 phényl)-2 quinoléinecarboxylate-4 de méthyle. On obtient alors 3,4 g de N,N-diéthyl (chloro-3 phényl)-2 quinoléinecarboxamide-4, que l'on transforme en son chlorhydrate en ajoutant à une solution du produit dans l'acétone une solution d'acide chlorhydrique dans l'oxyde diéthylique. Après recristallisation dans un mélange éthanol/oxyde diéthylique (2 : 1), ce chlorhydrate présente un point de fusion de 165°C.

Le (chloro-3 phényl)-2 quinoléinecarboxylate-4 de méthyle peut être préparé par action du méthanol sur l'acide (chloro-3 phényl)-2 quinoléinecarboxylique-4 en présence d'acide sulfurique concentré. Il présente un point de fusion de 101°C.

L'acide (chloro-3 phényl)-2 quinoléinecarboxylique-4 peut être préparé selon le procédé décrit par R.F. BROWN et al., J. Amer. Chem. Soc., 68, 2705 (1946).

**Exemple 37:** N,N-diéthyl (dichloro-3,4 phényl)-2 quinoléinecarboxamide-4

On procède comme à l'exemple 35, en partant de 5 ml de diéthylamine, 23 ml d'une solution 1,6M de butyllithium dans l'hexane et 4 g de (dichloro-3,4 phényl)-2 quinoléinecarboxylate-4 d'éthyle. Après 3 recristallisations du résidu dans l'isopropanol, on isole 2,5 g de N,N-diéthyl (dichloro-3,4 phényl)-2 quinoléinecarboxamide-4 fondant à 170°C.

Le (dichloro-3,4 phényl)-2 quinoléinecarboxylate-4 d'éthyle peut être préparé par action de l'éthanol sur l'acide (dichloro-3,4 phényl)-2 quinoléinecarboxylique-4 en présence d'acide sulfurique concentré. Il présente un point de fusion de 74°C.

L'acide (dichloro-3,4 phényl)-2 quinoléinecarboxylique-4 peut être préparé selon le procédé décrit par R.F. BROWN et al., J. Amer. Chem. Soc., 68, 2705 (1946).

**Exemple 38:** N,N-diéthyl (fluoro-2 phényl)-2 quinoléinecarboxamide-4

On procède comme à l'exemple 35, en partant de 27 ml de diéthylamine, 12,5 ml d'une solution 1,6M de butyllithium dans l'hexane et 2 g de (fluoro-2 phényl)-2 quinoléinecarboxylate-4 d'éthyle. Après recristallisation du résidu dans l'éther isopropylique, on isole 2 g de N,N-diéthyl (fluoro-2 phényl)-2 quinoléinecarboxamide-4 fondant à 92°C.

Le (fluoro-2 phényl)-2 quinoléinecarboxylate-4 d'éthyle peut être préparé par action de l'éthanol sur l'acide (fluoro-2 phényl)-2 quinoléinecarboxylique-4 en présence d'acide sulfurique concentré. Il présente un point de fusion de 86°C.

L'acide (fluoro-2 phényl)-2 quinoléinecarboxylique-4 peut être préparé selon le procédé décrit par R.F. BROWN et al. J. Amer. Chem. Soc., 68, 2705 (1946).

**Exemple 39:** {[(chloro-2 phényl)-2 quinolyl-4]carbonyl}-1 diméthyl-2,6 pipéridine.

On chauffe au reflux, pendant une heure, 5,7 g d'acide (chloro-2 phényl)-2 quinoléinecarboxylique-4 et 15 ml de chlorure de thionyle. On évapore le chlorure de thionyle, reprend le résidu par 40 ml de toluène, et évapore à nouveau. On ajoute alors au résidu obtenu 50 ml de toluène, puis on introduit goutte-à-goutte, sous agitation, 6,78 g de diméthyl-2,6 pipéridine. On agite une heure à température ambiante, filtre le précipité et évapore le filtrat sous pression réduite. Le résidu est repris par 50 ml d'eau et 50 ml de chloroforme. La phase organique est décantée et la phase aqueuse extraite par 50 ml de chloroforme. Les phases organiques sont rassemblées, séchées sur sulfate de magnésium et évaporées sous pression réduite.

On obtient, après recristallisation du résidu obtenu dans l'acétate d'éthyle, 3,8 g de {[(chloro-2 phényl)-2 quinolyl-4] carbonyl}-1 diméthyl-2,6 pipéridine fondant à 163°C.

**Exemple 40:** N,N-diisobutyl (chloro-2 phényl)-2 quinoléinecarboxamide-4

On chauffe au reflux, pendant une heure, 2,8 g d'acide (chloro-2 phényl)-2 quinoléinecarboxylique-4 dans 10 ml de chlorure de thionyle. On évapore le chlorure de thionyle, reprend le résidu par 40 ml de toluène et évapore à nouveau. On ajoute alors au résidu obtenu 20 ml de toluène et introduit goutte-à-goutte, sous agitation, une solution de 5,16 g de diisobutylamine dans 20 ml de toluène. On agite une nuit à température ambiante, évapore sous pression réduite, reprend le résidu par 50 ml de chloroforme et 100 ml d'eau. La phase organique est décantée, séchée sur sulfate de magnésium et évaporée sous pression réduite.

Le résidu obtenu est dissous dans 100 ml d'acétate d'éthyle, la phase organique est extraite par 100 ml d'une solution normale d'acide acétique, lavée par 4 fois 100 ml d'eau, séchée sur sulfate de magnésium et évaporée sous pression réduite. Le résidu obtenu est repris dans l'acétone et, après addition d'une solution d'acide chlorhydrique dans l'éther, on isole 1,5 g de chlorhydrate de N,N-diisobutyl (chloro-2 phényl)-2 quinolé inecarboxamide-4 fondant à 124°C.

**Exemple 41:** N-méthyl N-phényl phényl-2 quinoléinecarboxamide-4

A une solution de 12,8 g de N-méthylaniline dans 75 ml de chlorure de méthylène, refroidie à 0°C, on ajoute sous agitation, par portions, en 15 minutes, 10,7 g de chlorhydrate de chlorure de l'acide phényl-2 quinoléinecarboxylique-4. On agite 2 heures et trente minutes à 10°C. La solution obtenue est lavée par 120 ml d'une solution à 20 % d'acide acétique dans l'eau. La solution acétique est extraite par 100 ml de chlorure de méthylène. Les phases organiques sont rassemblées, lavées par 3 fois 100 ml d'eau, séchées sur sulfate de magnésium et évaporées sous pression réduite.

Après recristallisation du résidu dans l'acétonitrile, on obtient 9,1 g de N-méthyl N-phényl phényl-2 quinoléinecarboxamide-4 fondant à 141°C.

**Exemple 42:** N,N-diméthyl phényl-2 quinoléinecarboxamide-4

A une solution agitée de 18,9 g de N,N-diméthylamine dans 100 ml de chlorure de méthylène, refroidie à 0°C, on ajoute en une heure, par portions, 10,7 g de chlorhydrate du chlorure de l'acide phényl-2 quinoléinecarboxylique-4. On agite une heure à 0°C. La solution est évaporée sous pression réduite. Le résidu est repris dans 100 ml de chlorure de méthylène. La phase organique est lavée par 3 fois 100 ml d'eau, séchée sur sulfate de magnésium et évaporée sous pression réduite.

Après recristallisation du résidu dans l'acétonitrile, on isole 7,9 g de N,N-diméthyl phényl-2 quinoléinecarboxamide-4 fondant à 158°C.

**Exemple 43:** N-méthyl N-(méthyl-1 propyl) phényl-2 quinoléinecarboxamide-4

On opère comme à l'exemple 42, en partant de 10,7 g de chlorhydrate du chlorure de l'acide phényl-2 quinoléinecarboxylique-4, et de 10,5 g de N-méthyl butanamine-2 dans 75 ml de chlorure de méthylène.

Après recristallisation dans l'acétonitrile, on isole 10,1 g de N-méthyl N-(méthyl-1 propyl) phényl-2 quinoléinecarboxamide-4 fondant à 146°C.

**Exemple 44:** Phényl-2 [(tétrahydro-1,2,3,6 pyridyl-1) carbonyl]-4 quinoléine.

On procède comme à l'exemple 41, mais en partant de 10 g de tétrahydro-1,2,3,6 pyridine dans 75 ml de chlorure de méthylène et de 10,7 g de chlorhydrate du chlorure de l'acide phényl-2 quinoléinecarboxylique-4.

La base obtenue est dissoute dans l'oxyde diéthylique, puis transformée en son chlorhydrate par addition d'une solution d'acide chlorhydrique dans l'éthanol. Après recristallisation dans l'éthanol, on isole 7 g de phényl-2 [(tétrahydro-1,2,3,6 pyridyl-1)carbonyl]-4 quinoléine sous forme de chlorhydrate présentant une fusion pâteuse entre 130 et 140°C.

**Exemple 45:** N-benzyl N-méthyl phényl-2 quinoléinecarboxamide-4

On procède comme à l'exemple 41, mais en partant de 14,5 g de benzylméthylamine dans 100 ml de chlorure de méthylène et de 9,1 g de chlorhydrate du chlorure de l'acide phényl-2 quinoléinecarboxylique-4.

Après recristallisation dans l'acétonitrile, on isole 7 g de N-benzyl N-méthyl phényl-2 quinoléinecarboxamide-4 fondant à 106-110°C.

**Exemple 46:** N-cyclohexyl N-méthyl phényl-2 quinoléinecarboxamide-4

On procède comme à l'exemple 41, en partant de 9,1 g de chlorhydrate de chlorure de l'acide phenyl-2 quinoléinecarboxylique-4 et de 13,5 g de N-méthyl cyclohexylamine dans 100 ml de chlorure de méthylène.

Après recristallisation dans l'acétonitrile, on isole 9,3 g de N-cyclohexyl N-méthyl phényl-2 quinoléinecarboxamide-4 fondant à 168°C.

**Exemple 47**: N-éthyl N-[(pipéridyl-4) méthyl] phényl-2 quinoléinecarboxamide-4.

A une solution agitée de 9,1 g de chlorure de l'acide phényl-2 quinoléinecarboxylique-4 et de 8,4 ml de triéthylamine dans 100 ml de toluène, on ajoute lentement, à température ambiante, une solution de 7 g de N-éthyl(benzyl-1 pipéridyl-4) méthanamine dans 50 ml de toluène. On agite 1 heure à température ambiante. Le mélange réactionnel est coulé dans 100 ml d'eau, le toluène évaporé sous pression réduite. On ajoute alors 200 ml d'éther. La phase organique est décantée, la phase aqueuse extraite par 100 ml d'éther. Les phases organiques sont rassemblées, lavées par 2 fois 50 ml d'eau, puis extraitées par 3 fois 20 ml d'une solution aqueuse 2N d'acide chlorhydrique. La phase aqueuse acide est lavée à l'éther, amenée à pH 10 par addition d'une solution concentrée d'hydroxyde de sodium et extraite par 3 fois 150 ml d'éther. La phase éthérée est lavée par 3 fois 50 ml d'eau et concentrée sous pression réduite. On obtient alors 12 g de N-éthyl N-[(benzyl-1 pipéridyl-4) méthyl] phényl-2 quinoléinecarboxamide-4.

La débenzylation s'effectue de la façon suivante:

On chauffe jusqu'à dissolution 9,6 g de N-éthyl N-[(benzyl-1 pipéridyl-4) méthyl] phényl-2 quinoléinecarboxamide-4 dans 200 ml d'éthanol. Après refroidissement, on ajoute à cette solution 9,2 ml d'une solution éthanolique 4,5N d'acide chlorhydrique, 2,9 g de charbon palladié à 10 % de palladium comme catalyseur, et enfin 1,7 g d'acétate de sodium. On hydrogène sous agitation à 60°C, sous une pression d'hydrogène égale à la pression normale (1 bar), pendant 5 heures. Au bout de 5 heures (la quantité d'hydrogène absorbée est alors de 470 ml), on élimine le catalyseur par filtration, évapore l'éthanol par distillation sous pression réduite, reprend le résidu par 200 ml d'eau, amène à pH 10 par addition d'une solution concentrée d'hydroxyde de sodium, et extrait par 3 fois 100 ml d'éther. La phase organique est lavée par 3 fois 50 ml d'eau, séchée sur sulfate de magnésium et évaporée sous pression réduite. On isole ainsi 5 g de N-éthyl N-[(pipéridyl-4) méthyl] phényl-2 quinoléinecarboxamide-4, que l'on met en solution dans l'acétone et transforme en monochlorhydrate par addition d'une solution d'acide chlorhydrique dans l'éther. Ce monochlorhydrate présente un point de fusion de 219°C.

La N-éthyl (benzyl-1 pipéridyl-4) méthanamine peut être préparée par réduction du N-éthyl benzoyl-1 pipéridinecarboxamide-4 ($3.10^{-2}$ mole) par l'aluminohydrure de lithium ($6.10^{-2}$ mole) dans 100 ml de tétrahydrofuranne.

Le N-éthyl benzoyl-1 pipéridinecarboxamide-4 peut être préparé par action du chlorure de thionyle ($6,7.10^{-2}$ mole) sur l'acide benzoyl-1 pipéridinecarboxylique-4 ($4,5.10^{-2}$ mole) dans 100 ml de chloroforme et réaction du chlorure d'acide ainsi obtenu avac 0,22 mole d'éthylamine dans 30 ml de toluène.

L'acide benzoyl-1 pipéridinecarboxylique-4 peut être préparé par action du chlorure de benzoyle (0,85 mole) sur l'acide pipéridinecarboxylique-4 (0,85 mole) en présence d'une solution à 10 % de carbonate de potassium (3300 ml). Ce produit présente un point de fusion de 134°C.

**Exemple 48**: N-éthyl N-(pipéridyl-4) phényl-2 quinoléinecarboxamide-4

A une suspension agitée de 17,5 g de chlorure de l'acide phényl-2 quinoléinecarboxylique-4 dans 250 ml de toluène, on ajoute lentement 16 ml de triéthylamine, puis, en 30 minutes, une solution de 12,6 g de N-éthyl benzyl-1 pipéridinamine-4 dans 50 ml de toluène. On agite 4 heures à température ambiante.

Le milieu réactionnel est coulé dans 250 ml d'eau, le toluène évaporé par distillation sous vide, et on ajoute 200 ml d'oxyde diéthylique. La phase organique est décantée, la phase aqueuse est extraite par 2 fois 200 ml d'oxyde diéthylique. Les phases organiques sont rassemblées, lavées par 2 fois 50 ml d'eau, séchées sur sulfate de magnésium et concentrées par distillation sous pression réduite. Le résidu est chromatrographié sur du gel de silica en utilisant comme éluant un mélange toluène/diéthylamine (9 : 1). On obtient ainsi 25 g da N-éthyl N-(benzyl-1 pipéridyl-4) phényl-2 quinoléinecarboxamide-4.

La débenzylation s'effectue de la façon suivante:

A une solution agitée de 17 g de N-éthyl N-(benzyl-1 pipéridyl-4) phényl-2 quinoléinecarboxamide-4 dans 340 ml de toluène, on ajoute 8 g de chloroformiate de trichloro-2,2,2,éthyle. On chauffe 6 heures au reflux, ajoute encore 1,6 g de chloroformiate de trichloro-2,2,2 éthyle et chauffe 2 heures au reflux. Le mélange réactionnel est coulé dans 300 ml d'eau, alcalinisé à l'aide d'une solution aqueuse 2N d'hydroxyde de sodium, et extrait par 3 fois 150 ml d'acétate d'éthyle. La phase organique est lavée par 2 fois 50 ml d'eau, séchée sur sulfate de magnésium, et concentrée sous pression réduite.

A l'huile obtenue on ajoute 450 ml d'éthanol, puis 200 ml d'une solution aqueuse 6N d'hydroxyde de potassium. On agite quelques minutes, puis on chauffe au reflux sous agitation pendant 6 heures. Après évaporation de l'éthanol sous pression réduite, le mélange réactionnel est repris par 300 ml d'eau, puis par 180 ml d'une solution aqueuse 6N d'acide chlorhydrique, et extrait enfin par 3 fois 200 ml de chloroforme. La phase organique est lavée par 2 fois 50 ml d'eau, séchée sur sulfate de magnésium et évaporée sous pression réduite. Le résidu est chromatographié sur du gel de silice en utilisant comme éluant un mélange toluène/méthanol/diéthylamine (9 : 1 : 1). On isole ainsi 3,3 g de N-éthyl N-(pipéridyl-4) phényl-2 quinoléinecarboxamide-4.

Ce composé est dissous dans de l'éthanol, puis transformé en son monochlorhydrate par addition d'une

solution d'acide chlorhydrique dans l'éther. Le monochlorhydrate présente un point de fusion supérieur à 250°C.

La N-éthyl benzyl-1 pipéridinamine-4 peut être préparée par réduction de la N-acétyl benzyl-1 pipéridinamine-4 ($6,4.10^{-2}$ mole) par de l'aluminohydrure de lithium (0,128 mole) dans 200 ml de tétrahydrofuranne. La N-acétyl benzyl-1 pipéridineamine-4 peut être préparée par action de chlorure d'acétyle ($1,15.10^{-1}$ mole) sur la benzyl-1 pipéridineamine-4 ($1,05.10^{-1}$ mole) dans 100 ml de chloroforme en présence de triéthylamine ($1,05.10^{-1}$ mole).

**Exemple 49:** N-éthyl N-[(pipéridyl-4)-2 éthyl] phényl-2 quinoléinecarboxamide-4.

A une suspension agitée de 8,3 g de chlorure de l'acide phényl-2 quinoléinecarboxylique-4 dans 50 ml de toluène, on ajoute 6,7 ml de triéthylamine, puis, en 30 minutes, une solution de 6 g de N-éthyl (benzyl-1 pipéridyl-4) éthanamine dans 50 ml de toluène. On agite 20 heures à température ambiante. On coule le mélange réactionnel dans 100 ml d'eau, évapore le toluène par distillation sous pression réduite, reprend par 200 ml d'oxyde diéthylique, ajoute 0,8 ml de triéthylamine. La phase organique est décantée, la phase aqueuse extraite par 2 fois 100 ml d'oxyde diéthylique. Les phases organiques sont rassemblées, lavées par 2 fois 30 ml d'eau, séchées sur sulfate de magnésium et évaporées sous pression réduite. Le résidu est chromatographié sur du gel de silice en utilisant comme éluant un mélange cyclohexane/diéthylamine (9 : 1). On obtient ainsi 9,7 g de N-éthyl N-[(benzyl-1 pipéridyl-4)-2 éthyl] phényl-2 quinoléinecarboxamide-4.

La débenzylation s'effectue de la façon suivante:

A une solution agitée de 5,8 g de N-éthyl N-[(benzyl-1 pipéridyl-4)-2 éthyl] phényl-2 quinoléinecarboxamide-4 dans 100 ml de toluène, on ajoute 2,6 g de chloroformiate de trichloro-2,2,2 éthyle. On chauffe 1 heure au reflux. Le mélange réactionnel est coulé dans 100 ml d'eau, alcalinisé à l'aide d'une solution aqueuse 2N d'hydroxyde de sodium et extrait par 3 fois 100 ml d'acétate d'éthyle. La phase organique est lavée par 2 fois 30 ml d'eau, séchée sur sulfate de magnésium et évaporée sous pression réduite.

A l'huile obtenue, on ajoute 150 ml d'éthanol, puis, sous agitation, 64 ml d'une solution aqueuse 6N d'hydroxyde de potassium. On chauffe 2 heures au reflux, puis agite 15 heures à température ambiante. On ajoute au milieu réactionnel 100 ml d'eau, évapore l'éthanol par distillation sous pression réduite, ajoute encore 100 ml d'eau, puis extrait par 3 fois 150 ml d'oxyde diéthylique et enfin par 150 ml de chlorure de méthylène. Les phases organiques sont rassemblées, extraites par 4 fois 25 ml d'une solution aqueuse N d'acide acétique. La phase acide acétique est amenée à pH 10 par addition d'une solution aqueuse 2N d'hydroxyde de sodium et extraite à nouveau par 3 fois 50 ml de chlorure de méthylène. La phase organique est lavée par 30 ml d'eau, séchée sur sulfate de magnésium et évaporée sous pression réduite. Le résidu est chromatographié sur du gel de silice en utilisant comme éluant un mélange toluène/diéthylamine (9 : 1). On isole ainsi 2,2 g de N-éthyl N-[(pipéridyl-4)-2 éthyl] phényl-2 quinoléinecarboxamide-4. Ce composé est transformé au sein de l'éthanol en son monochlorhydrate. Celui-ci présente un point de fusion égal à 210°C.

La N-éthyl (benzyl-1 pipéridyl-4) éthanamine peut être préparée par réduction du N-éthyl(benzoyl-1 pipéridyl-4) acétamide ($2,9.10^{-2}$ mole) par l'aluminohydrure de lithium ($5,8.10^{-2}$ mole) dans 100 ml de tétrahydrofuranne.

Le N-éthyl (benzoyl-1 pipéridyl-4) acétamide peut être préparé par action du chlorure de thionyle ($6.10^{-2}$ mole) sur l'acide (benzoyl-1 pipéridyl-4) acétique ($4,10^{-2}$ mole) dans 100 ml de chloroforme et action du chlorure d'acide ainsi obtenu sur 0,2 mole d'éthylamine dans 130 ml de toluène.

L'acide (benzoyl-1 pipéridyl-4) acétique peut être préparé par action du chlorure de benzoyle ($1,2.10^{-1}$ mole) sur le sel de potassium de l'acide (pipéridyl-4) acétique ($10^{-1}$ mole) à 0°C dans l'eau (100 ml). Il présente un point de fusion de 137°C.

**Exemple 50:** N-éthyl N-[(pipéridyl-4)-3 propyl) phényl-2 quinoléinecarboxamide-4.

On opère comme à l'exemple 48, en partant de 32 g de chlorure de l'acide phényl-2 quinoléinecarboxylique-4 dans 250 ml de toluène, de 12,3 ml de triéthylamine et de 11,4 g de N-éthyl (benzyl-1 pipéridyl-4) propanamine dans 50 ml de toluène. On obtient ainsi 21 g de N-éthyl N-[(benzyl-1 pipéridyl-4)-3 propyl] phényl-2 quinoléinecarboxamide-4.

La débenzylation s'effectue comme à l'exemple 48, en partant de 21 g de N-éthyl N-[(benzyl-1 pipéridyl-4)-3 propyl) phényl-2 quinoléinecarboxamide-4 dans 400 ml de toluène, de 9 g de chloroformiate de trichloro-2,2,2 éthyle, puis de 245 ml d'une solution aqueuse 6N d'hydroxyde de potassium et de 550 ml d'éthanol. Après chromatographie du résidu sur du gel de silice en utilisant comme éluant un mélange toluène/diéthylamine (9 : 1), on isole 10 g de N-éthyl N-[(pipéridyl-4)-3 propyl] phényl-2 quinoléinecarboxamide-4, que l'on transforme, au sein de l'éthanol, en son méthanesulfonate par addition d'une solution éthanolique d'acide méthanesulfonique. Le méthanesulfonate présente un point de fusion de 164°C.

La N-éthyl (benzyl-1 pipéridyl-4) propanamine peut être préparée par réduction du N-éthyl (benzoyl-1 pipéridyl-4) propionamide ($3,4.10^{-2}$ mole) par de l'aluminohydrure de lithium ($6,9.10^{-2}$ mole) dans 200 ml de tétrahydrofuranne.

L. N-éthyl (benzoyl-1 pipéridyl-4) propionamide peut être préparé par action du chlorure de thionyle ($8,6.10^{-2}$ mole) sur l'acide (benzoyl-1 pipéridyl-4) propionique dans 150 ml de chloroforme, et action du chlorure d'acide ainsi obtenu sur 2,85 mole d'éthylamine dans 150 ml de toluène.

L'acide N-benzoyl pipéridine-4 propionique peut être préparé selon le procédé décrit par KOELSCH (J. Amer.

Chem. Soc., 1943, 65, 2460).

**Exemple 51**: N-méthyl N-(méthyl-1 propyl) phényl-3 isoquinoléinecarboxamide-1.

A 2,4 g d'acide phényl-3 isoquinoléinecarboxylique-1 dans 100 ml de chloroforme, on ajoute 1,1 g de triéthylamine. On refroidit à 10°C et ajoute 1,21 g de chloroformiate d'éthyle. Après 40 minutes d'agitation, on ajoute 1,1 g de N-méthyl butanamine-2, et agite 4 heures à température ambiante. On lave la phase organique avec une solution aqueuse saturée de carbonate de sodium, la sèche sur du sulfate de magnésium et l'évapore sous pression réduite jusqu'à siccité. On chromatographie le résidu sur du gel de silice avec un mélange cyclohexane/acétate d'éthyle (7 : 3) comme éluant et recristallise le produit récupéré dans de l'éther isopropylique. On obtient ainsi 1,36 g de N-méthyl N-(méthyl-1 propyl) phényl-3 isoquinoléinecarboxamide-1, fondant à 125°C après recristallisation dans l'éther isopropylique.

L'acide phényl-3 isoquinoléinecarboxylique-1 peut être obtenu à partir de la méthyl-1 phényl-3 isoquinoléine. Au moyen de la N-bromosuccinimide on forme tout d'abord la dibromométhyl-1 phényl-3 isoquinoléine que l'on oxyde ensuite avec du nitrate d'argent d'abord en milieu neutre, puis en milieu basique. Cet acide a un point de fusion de 134°C.

**Exemple 52**: [(Phényl-2 quinazolinyl-4) carbonyl]-1 pipéridine-4 éthanol.

On chauffe à 150°C pendant 4 heures 1,7 g de phényl-2 quinazolinecarboxylate-4 d'éthyle et 8 g de pipéridine-4 éthanol. Après refroidissement, on ajoute de l'eau et extrait à l'acétate d'éthyle. On sèche la phase organique sur du sulfate de magnésium et l'évapore à sec sous pression réduite. On chromatrographie le résidu sur du gel de silice avec de l'acétate d'éthyle comme éluant et recristallise le produit récupéré dans l'éther isopropylique. On obtient 0,5 g de [(phényl-2 quinazolinyl-4) carbonyl]-1 pipéridine-4 éthanol fondant à 146°C.

**Exemple 53**: [(phényl-2 quinazolinyl-4) carbonyl]-1 pipéridinone-4

Sous atmosphère d'azote et à 0°C, on ajoute 37 ml d'une solution 1,6M de butyllithium dans l'hexane à 7,5 ml de dioxa-1,4 aza-8 spiro-[4,5] décane dans 30 ml de tétrahydrofuranne anhydre. Après 30 minutes, on ajoute 8 g de phényl-2 quinazolinecarboxylate-4 d'éthyle en solution dans 30 ml de tétrahydrofuranne anhydre. Après 2 heures à la température ambiante, on ajoute de l'eau et extrait au chlorure de méthylène. On lave la phase organique à l'eau, la sèche sur du sulfate de magnésium et l'évapore à sec sous pression réduite. On reprend le résidu dans 100 ml d'acétone et 20 ml d'acide chlorhydrique concentré et agite 5 heures à la température ambiante.

On dilue avec de l'eau, extrait la phase aqueuse avec du chlorure de méthylène, lave la phase organique avec une solution normale d'hydroxyde de sodium puis avec de l'eau, la sèche sur du sulfate de magnésium et l'évapore à sec sous pression réduite. On obtient 1 g de produit que l'on chromatographie sur du gel de silice avec un mélange toluène/diéthylamine (95 : 5) comme éluant. On récupère 4,5 g de produit que l'on recristallise dans un mélange cyclohexane/acétate éthyle (1:1). On obtient ainsi 2,3 g de [(phényl-2 quinazolinyl-4) carbonyl]-1 pipéridinone-4 fondant à 161°C.

**Exemple 54**: N,N-diéthyl phényl-2 trifluorométhyl-8 quinoléinecarboxamide-4.

On procède comme à l'exemple 4, en partant de 10 g d'acide phényl-2 trifluorométhyl-8 quinoléinecarboxylique-4, de 30 ml de chlorure de thionyle et de 16,2 ml de diéthylamine.

Après chromatographie sur du gel de silice avec un mélange cyclohexane/acétate d'éthyle (70 : 30) comme éluant et recristallisation dans un mélange éther isopropylique/éther de pétrole (8 : 3), on isole 9,2 g de N,N-diéthyl phényl-2 trifluorométhyl-8 quinoléinecarboxamide-4 fondant à 114°C.

L'acide phényl-2 trifluorométhyl-8 quinoléinecarboxylique-4 peut être préparé selon le procédé décrit per D.W. BOYKIN et al., J. Med. Chem., 11 (2), 273-277 (1968).

**Propriétés pharmacollogiques**

Affinité pour les sites récepteurs des benzodiazépines

Cette affinité est mesurée par l'aptitude des produits à déplacer le diazépam tritié ($^3$H-diazépam) de son site de liaison et est exprimée par une valeur $K_i$, en micromoles (µM), qui est calculée par la formule:

$$K_i = \frac{IC_{50}}{1 + \frac{C}{K_D}}$$

dans laquelle C représente la concentration de $^{3}H$-diazépam utilisée, $K_D$ une constante d'affinité caractéristique du diazépam et $IC_{50}$ la concentration de produit nécessaire pour obtenir une inhibition de 50 % de la liaison du $^{3}H$-diazépam.

L'affinité des composés de formule (I) pour les sites récepteurs cérébraux des benzodiazépines a été déterminée selon la méthode de MOHLER et Coll., Life Sciences, 1977, 20, 2101, sur des membranes de cerveau de rat.

L'affinité des composés de formule (I) pour les sites récepteurs de type périphérique a été déterminée, en utilisant le protocole de BRAESTRUP et Coll., Proc. Natl. Acad. Sci. U.S.A., 1977, 74, 3805, sur des membranes de rein de rat,

A titre d'exemple, on a obtenu les résultats suivants:

Affinité pour les sites récepteurs de type cérébral

| Produits | $K_i$ (µM) |
|---|---|
| Exemple 3 | 0,16 |
| Exemple 4 | 0,04 |
| Exemple 5 | 0,09 |
| Exemple 10 | 0,21 |
| Exemple 11 | 0,13 |
| Exemple 24 | 0,33 |
| Exemple 25 | 0,39 |
| Exemple 26 | 0,09 |
| Exemple 27 | 0,07 |
| Exemple 38 | 0,31 |
| Exemple 49 | 0,40 |
| Exemple 53 | 0,27 |
| Chlordiazépoxide | 0,08 |

Affinité pour les sites récepteurs de type périphérique

| Produits | $K_i$ (µM) |
|---|---|
| Exemple 8 | 0,117 |
| Exemple 10 | 0,160 |
| Exemple 15 | 0,031 |
| Exemple 16 | 0,072 |
| Exemple 17 | 0,009 |
| Exemple 18 | 0,360 |
| Exemple 20 | 0,002 |
| Exemple 21 | 0,058 |
| Exemple 22 | 0,270 |
| Exemple 37 | 0,117 |
| Exemple 39 | 0,054 |
| Exemple 43 | 0,100 |
| Exemple 51 | 0,045 |
| Diazépam | 0,043 |
| Ro-5-4864 | 0,004 |

**Propriétés toxicologiques**

Les toxicités aiguës des composés de formule (I) ont été déterminées chez la souris mâle CD1 (Charles RIVER) par voie orale. Les $DL_{50}$ ont été calculées, après 3 jours d'observation, par la méthode cumulative de J.J. REED et H. MUENCH (Amer. J. Hyg. 1938, 27, 493).

Les composés de formule (I) se comportent comme des substances relativement peu toxiques chez la souris, puisque les $DL_{50}$ des composés se situent entre 200 et 1 000 mg/kg.

**Utilisation thérapeutique**

Les médicaments selon l'invention qui contiennent un composé de formule (I) ou un mélange de composés stéréoisomères répondant à la formule (I) ou un sel d'un tel composé ou mélange de composés stéréoisomères avec un acide pharmaceutiquement acceptable, associé à un véhicule pharmaceutiquement acceptable, sont utilisés en thérapeutique humaine pour le traitement des états d'anxiété et des troubles pulmonaires, rénaux,

cardiovasculaires ou circulatoires. Ils se présentent sous toutes les formes en usage dans le domaine des médicaments, telles que comprimés, capsules, gélules, suppositoires, solutions ingérables ou injectable.

La posologie dépend des effets recherchés et de la voie d'administration utilisée. Par exemple, par voie orale, elle est comprise entre 10 et 500 mg de substance active par jour, avec des doses unitaires allant de 2 à 100 mg de substances active.

Parmi les troubles pulmonaires, rénaux, cardiovasculaires ou circulatoires on peut citer l'hypertension, l'épilepsie, l'angor.

**Revendications** pour les Etats contractants BE, CH, DE, FR, GB, IT, LI, LU, NL, SE.

1. Médicaments contenant une substance active et un véhicule pharmaceutiquement acceptable, caractérisés en ce que la substance active est un composé répondant à la formule:

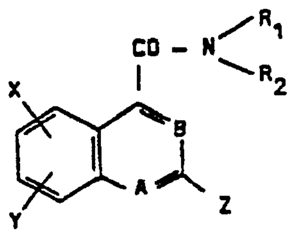

(I)

dans laquelle $R_1$ représente un groupe alkyle linéaire ou ramifié ayant 1 à 6 atomes de carbone, un groupe phényle, un groupe cycloalkyle ayant 3 à 6 atomes de carbone, un groupe phénylalkyle ou cycloalkylalkyle dont la partie alkyle comporte 1 à 3 atomes de carbone, ou un groupe

$$-\underset{R_5}{\overset{R_3}{\overset{|}{\underset{|}{C}}}}-R_4$$

dans lequel $R_3$ et $R_4$ sont des atomes d'hydrogène ou des groupes alkyle et $R_5$ est un groupe alcényle ou alcinyle, le somme des atomes de carbone de $R_3$, $R_4$ et $R_5$ étant de 2 à 5, $R_2$ représente un groupe alkyle linéaire ou ramifié ayant 1 à 6 atomes de carbone, un groupe phényle, un groupe cycloalkyle ayant 3 à 6 atomes de carbone, un groupe phénylalkyle ou cycloalkylalkyle dont la partie alkyle comporte 1 à 3 atomes de carbone, un groupe

$$-\underset{R_5}{\overset{R_3}{\overset{|}{\underset{|}{C}}}}-R_4$$

dans lequel $R_3$, $R_4$ et $R_5$ sont tels que définis ci-dessus, ou un groupe — $(CH_2)_n$— 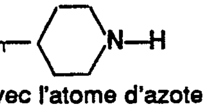

dans lequel n est 0, 1, 2 ou 3, $R_1$ et $R_2$ pouvant en outre former ensemble avec l'atome d'azote auquel ils sont rattachés un radical hétérocyclique à 5, 6 ou 7 chaînons pouvant contenir un autre hétéroatome choisi parmi l'azote et l'oxygène et pouvant porter un ou deux substituants choisis parmi les groupes alkyle ayant 1 à 3 atomes de carbone, le groupe hydroxy, le groupe oxo et les groupes hydroxyalkyle, diméthylaminoalkyle et diéthylaminoalkyle dont la partie alkyle comporte 1 à 3 atomes de carbone,

Z représente un groupe phényle, pyridyle, thiényle, thiazolyl-2, ou un groupe phényle substitué par un ou deux substituants pris parmi les atomes d'halogène, les groupes alkyle, alkoxy et alkylthio ayant 1 à 3 atomes de carbone, le groupe trifluorométhyle et le groupe nitro,

X et Y sont identiques ou différents et représentent des atomes d'hydrogène ou d'halogène, des groupes alkyle ou alkoxy ayant 1 à 3 atomes de carbone, des groupes nitro ou trifluorométhyle,

A représente un atome d'azote ou un groupe CH et

B représente un atome d'azote ou un groupe CH, à l'exception du N,N-diéthyl phényl-2 quinoléine carboxamide-4, ou un mélange de composés stéréoisomères répondant à la formule (I), ou un sel d'un tel composé ou mélange de composés stéréoisomères avec un acide pharmaceutiquement acceptable.

2. Médicaments selon la revendication 1, caractérisés en ce que, dans la formule (I), X et Y sont des atomes d'hydrogène.

3. Médicaments selon l'une des revendications 1 ou 2, caractérisés en ce que dans la formule (I) 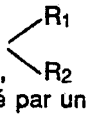 représente un radical pyrrolidinyle, piperidino, morpholino, pipérazinyle, N-méthyl pipérazinyle, tétrahydro-1,2, 3,6 pyridyle, hexahydro-2,3, 4,5, 6,7 azépinyle, oxo-4 pipéridino, pipéridino substitué par un ou deux groupes alkyle ayant 1 à 3 atomes de carbone ou par un groupe hydroxy en position 3 ou 4 ou encore par un groupe hydroxyalkyle, diméthylaminoalkyle ou diéthylaminoalkyle dont la partie alkyle a 1 à 3 atomes de carbone.

4. Médicaments selon la revendication 2, caractérisés en ce que, dans la formule (I) X et Y sont des atomes d'hydrogène, A est un atome d'azote, B est un atome d'azote ou un groupe CH, Z est le groupe phényle et

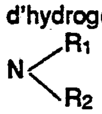

est un groupe diéthylamino, pipéridino ou pyrrolidino.

5. Médicaments selon la revendication 1, caractérisés en ce que la substance active est le N,N-diéthyl phényl-3 naphtalènecarboxamide-1 ou un composé de formule (I) selon la revendication 1 pour lequel X et Y sont des atomes d'hydrogène, Z est un groupe phényle, chloro-2 phényle ou chloro-3 phényle et au moins l'un des substituants $R_1$ et $R_2$ est un groupe alkyle ayant 3 à 6 atomes de carbone ramifié en position α par rapport à l'atome d'azote, ou un sel d'un tel composé avec un acide pharmaceutiquement acceptable.

6. Composés, racémiques ou stéréoisomères, répondant à la formule (I) de la revendication 1, dans laquelle soit A et B sont des atomes d'azote et X, Y, Z, $R_1$ et $R_2$ ont les significations indiquées à la revendication 1,

soit A est un groupe CH, B est un atome d'azote et X, Y, Z, $R_1$ et $R_2$ ont les significations indiquées à la revendication 1,

soit A et B sont des groupes CH, $R_1$ est un groupe alkyle linéaire ou ramifié 2-6C, phényle, cycloalkyle 3-6C, phénylalkyle ou cycloalkylalkyle dont la partie alkyle a 1 à 3 atomes de carbone, ou

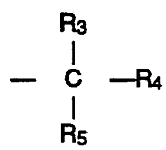

$R_3$, $R_4$ et $R_5$ ayant les significations indiquées à la revendication 1, $R_2$ est un groupe alkyle linéaire ou ramifié 1-6C, phényle, cycloalkyle 3-6C, phénylalkyle ou cycloalkylalkyle dont la partie alkyle a 1 à 3 atomes de carbone,

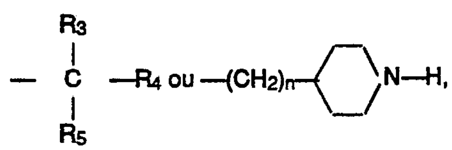

$R_3$, $R_4$, $R_5$ et n ayant les significations indiquées à la revendication 1, $R_1$ et $R_2$ pouvant en outre former ensemble avec l'atome d'azote auquel ils sont rattachés un radical hétérocyclique à 5, 6 ou 7 chaînons pouvant contenir un autre hétéroatome choisi parmi l'azote et l'oxygène et pouvant porter un ou deux substituants choisis parmi les groupes alkyle 1-3C, hydroxy, oxo, hydroxyalkyle, diméthylaminoalkyle et diethylaminoalkyle dont la partie alkyle a 1 à 3 atomes de carbone, et Z, X et Y ont les significations indiquées à la revendication 1,

soit A est un atome d'azote, B est un groupe CH, Z, X et Y ont les significations indiquées à la revendication 1, $R_1$ est un groupe phényle, cycloalkyle 3-6C, phénylalkyle ou cycloalkylalkyle dont la partie alkyle a 1 à 3 atomes de carbone, ou

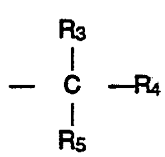

$R_3$, $R_4$ et $R_5$ ayant les significations indiquées à la revendication 1, $R_2$ est un groupe phényle, cycloalkyle 3-6C, phenylalkyle ou cycloalkylalkyle dont la partie alkyle a 1 à 3 atomes de carbone,

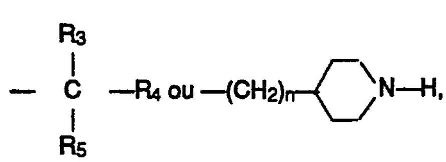

$R_3$, $R_4$, $R_5$ et n ayant les significations indiquées à la revendication 1, $R_1$ et $R_2$ pouvant en outre former ensemble

avec l'atome d'azote auquel ils sont rattachés un radical pyrrolidinyle, pipéridino, morpholino, tétrahydro-1,2,3,6 pyridyle, hexahydro-2,3,4,5,6,7 azépinyle, oxo-4 pipéridino ou pipéridino substitué par un ou deux groupe alkyle 1-3C ou par un groupe hydroxy en position 3 ou 4 ou par un groupe hydroxyalkyle, diméthylaminoalkyle ou diéthylaminoalkyle dont la partie alkyle a 1 à 3 atomes de carbone, $R_1$ et $R_2$ peuvent aussi être des groupes alkyle différents 1-6C.

7. Composés selon la revendication 6 pour lesquels X et Y sont des atomes d'hydrogène.

8. Composés selon la revendication 6 pour lesquels soit A est un groupe CH et B est un atome d'azote, soit A et B sont des groupes CH, soit A est un atome d'azote et B est un groupe CH et 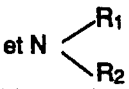 représente un radical pyrrolidinyle, pipéridino, morpholino, pipérazinyle, N-méthyl pipérazinyle, tétrahydro-1,2,3,6 pyridyle, hexahydro-2,3,4,5,6,7 azépinyle, oxo-4 pipéridino, pipéridino substitué par un ou deux groupes alkyle ayant 1 à 3 atomes de carbone ou par un groupe hydroxy en position 3 ou 4 ou encore par un groupe hydroxyalkyle, diméthylaminoalkyle ou diéthylaminoalkyle dont la partie alkyle a 1 à 3 atomes de carbone.

9. Procédé de préparation des composés selon la revendication 6, caractérisé en ce que l'on fait réagir une amine de formule 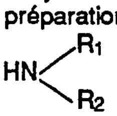
sur un composé de formule:

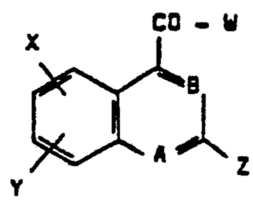

X, Y, Z, A, B, $R_1$ et $R_2$ ayant dans les formules ci-dessus les mêmes significations que dans la revendication 6 et W représentant un groupe alkoxy de bas poids moléculaire, un atome de chlore ou un groupe alcoxycarbonyloxy de bas poids moléculaire.

10. Procédé de préparation des composés selon la revendication 6, pour lesquels $R_2$ est un groupe

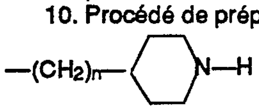

caractérisé en ce que l'on fait réagir une amine de formule:

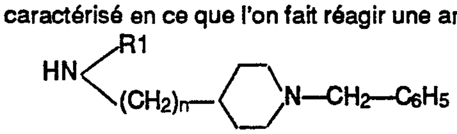

sur un composé de formule:

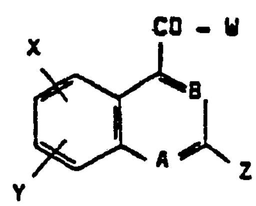

X, Y, Z, A, B, $R_1$ et n ayant dans les formules ci-dessus les mêmes significations que dans la revendication 6 et W représentant un groupe alcoxy de bas poids moléculaire, un atome de chlore ou un groupe alcoxycarbonyloxy de bas poids moléculaire, et soumet le composé ainsi obtenu à une débenzylation.

**Revendications** pour l'Etat contractant AT.

1. Procédé de préparation des composés racémiques ou stéréoisomères de formule:

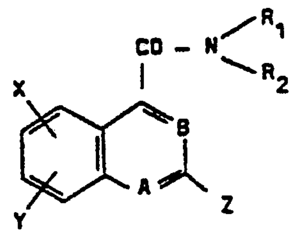

(I)

dans laquelle soit A et B sont des atomes d'azote et $R_1$ représente un groupe alkyle linéaire ou ramifié ayant 1 à 6 atomes de carbone, un groupe phényle, un groupe cycloalkyle ayant 3 à 6 atomes de carbone, un groupe phénylalkyle ou cycloalkylalkyle dont la partie alkyle comporte 1 à 3 atomes de carbone, ou un groupe

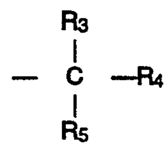

dans lequel $R_3$ et $R_4$ sont des atomes d'hydrogène ou des groupes alkyle et $R_5$ est un groupe alcényle ou alcinyle, la somme des atomes de carbone de $R_3$, $R_4$ et $R_5$ étant de 2 à 5, $R_2$ représente un groupe alkyle linéaire ou ramifié ayant 1 à 6 atomes de carbone, un groupe phényle, un groupe cycloalkyle ayant 3 à 6 atomes de carbone, un groupe phénylalkyle ou cycloalkylalkyle dont la partie alkyle comporte 1 à 3 atomes de carbone, un groupe

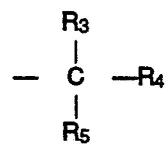

dans lequel $R_3$, $R_4$ et $R_5$ sont tels que définis ci-dessus, ou un groupe $-(CH_2)_n-$ 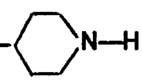

dans lequel n est 0, 1, 2 ou 3, $R_1$ et $R_2$ pouvant en outre former ensemble avec l'atome d'azote auquel ils sont rattachés un radical hétérocyclique à 5, 6 ou 7 chaînons pouvant contenir un autre hétéroatome choisi parmi l'azote et l'oxygène et pouvant porter un ou deux substituants choisis parmi les groupes alkyle ayant 1 à 3 atomes de carbone, le groupe hydroxy, le groupe oxo et les groupes hydroxyalkyle, diméthylaminoalkyle et diéthylaminoalkyle dont la partie alkyle comporte 1 à 3 atomes de carbone,

Z représente un groupe phényle, pyridyle, thiényle, thiazolyl-2, ou un groupe phényle substitué par un ou deux substituants pris parmi les atomes d'halogène, les groupes alkyle, alkoxy et alkylthio ayant 1 à 3 atomes de carbone, le groupe trifluorométhyle et le groupe nitro,

X et Y sont identiques ou différents et représentent des atomes d'hydrogène ou d'halogène, des groupes alkyle ou alkoxy ayant 1 à 3 atomes de carbone, des groupes nitro ou trifluorométhyle,

soit A est un groupe CH, B est un atome d'azote et X, Y, Z, $R_1$ et $R_2$ ont les significations indiquées ci-dessus;

soit A et B sont des groupes CH, $R_1$ est un groupe alkyle linéaire ou ramifié 2-6C, phényle, cycloalkyle 3-6C, phénylalkyle ou cycloalkylalkyle dont la partie alkyle a 1 à 3 atomes de carbone, ou

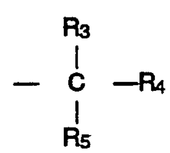

ou $R_3$, $R_4$ et $R_5$ ayant les significations indiquées ci-dessus $R_2$ est un groupe alkyle linéaire ou ramifié 1-6C, phényle, cycloalkyle 3-6C, phénylalkyle ou cycloalkylalkyle dont la partie alkyle a 1 à 3 atomes de carbone,

ou $-(CH_2)_n-$ 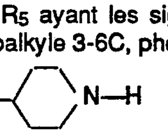

$R_3$, $R_4$, $R_5$ et n ayant les significations indiquées ci-dessus $R_1$ et $R_2$ pouvant en outre former ensemble avec l'atome d'azote auquel ils sont rattachés un radical hétérocyclique à 5, 6 ou 7 chaînons pouvant contenir un autre hétéroatome choisi parmi l'azote et l'oxygène et pouvant porter un ou deux substituants choisis parmi les groupes alkyle 1-3C, hydroxy, oxo, hydroxyalkyle, diméthylaminoalkyle et diéthylaminoalkyle dont la partie alkyle a 1 à 3 atomes de carbone, et Z, X et Y ont les significations indiquées ci-dessus;

soit A est un atome d'azote, B est un groupe CH, Z, X et Y ont les significations indiquées ci-dessus, $R_1$ est

un groupe phényle, cycloalkyle 3-6C, phénylalkyle ou cycloalkylalkyle dont la partie alkyle a 1 à 3 atomes de carbone, ou

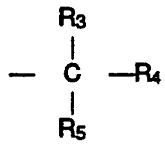

$R_3$, $R_4$ et $R_5$ ayant les significations indiquées ci-dessus, $R_2$ est un groupe phényle cycloalkyle 3-6C, phénylalkyle ou cycloalkylalkyle dont la partie alkyle a 1 à 3 atomes de carbone,

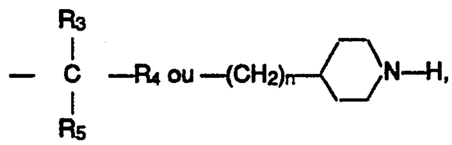

$R_3$, $R_4$, $R_5$ et n ayant les significations indiquées ci-dessus, $R_1$ et $R_2$ pouvant en outre former ensemble avec l'atome d'azote auquel ils sont rattachés un radical pyrrolidinyle, pipéridino, morpholino, tétrahydro-1,2,3,6 pyridyle, hexahydro-2,3,4,5,6,7 azépinyle, oxo-4 pipéridino ou pipéridino substitué par un ou deux groupes alkyle 1-3C ou par un groupe hydroxy en position 3 ou 4 ou par un groupe hydroxyalkyle, diméthylaminoalkyle ou diéthylaminoalkyle dont la partie alkyle a 1 à 3 atomes de carbone, ou $R_1$ et $R_2$ peuvent être des groupes alkyle différents, 1-6C caractérisé en ce que l'on fait réagir une amine de formule 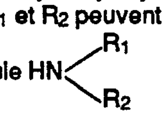

sur un composé de formule:

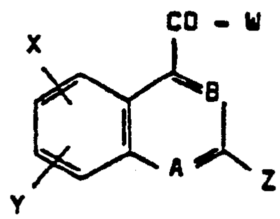

X, Y, Z, A, B, $R_1$ et $R_2$ ayant les mêmes significations que ci-dessus et W représentant un groupe alkoxy de bas poids moléculaire, un atome de chlore ou un groupe alcoxycarbonyloxy de bas poids moléculaire.

2. Procédé selon la revendication 1 pour la préparation des composés de formule (I) dans laquelle $R_2$ est un groupe —(CH2)n— 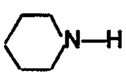

caractérisé en ce que l'on fait réagir une amine de formule:

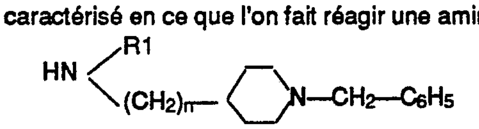

sur un composé de formule:

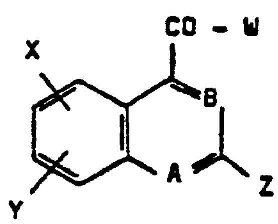

X, Y, Z, A, B, $R_1$ et n ayant dans les formules ci-dessus les mêmes significations que dans la revendication 1 et W représentant un groupe alcoxy de bas poids moléculaire, un atome de chlore ou un groupe alcoxycarbonyloxy de bas poids moléculaire, et soumet le composé ainsi obtenu à une débenzylation.

3. Procédé selon l'une des revendications 1 ou 2 pour la préparation de composés de formule (I) dans laquelle X et Y sont des atomes d'hydrogène.

4. Procédé selon la revendication 1 pour la préparation de composés de formule (I) dans laquelle X et Y sont des atomes d'hydrogène, A est un atome d'azote, B est un atome d'azote ou un groupe CH, Z est le groupe phényle et 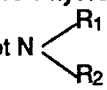 est un groupe diéthylamino, pipéridino ou pyrrolidino.

5. Procédé selon la revendication 1 pour la préparation de composés de formule (I) dans laquelle X et Y sont des atomes d'hydrogène Z est un groupe phényle, chloro-2 ou 3 phényle et au moins l'un des substituants $R_1$ et $R_2$ est un groupe alkyle ayant 3 à 6 atomes de carbone ramifié en position α par rapport à l'atome d'azote, A et B sont comme définis dans la revendication 1.

6. Procédé selon la revendication 1 pour la préparation des composés de formule (I) dans laquelle soit A est un groupe CH et B est un atome d'azote, soit A et B sont des groupes CH, soit A et B sont des atomes d'azote et

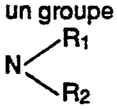

représente un radical pyrrolidinyle, piperidino, morpholino, pipérazinyle, N-méthylpipérazinyle, tétrahydro-1,2,3,6 pyridyle, hexahydro-2,3,4,5,6,7 azépinyle, oxo-4 pipéridino, pipéridino substitué par un ou deux groupes alkyle ayant 1 à 3 atomes de carbone ou par un groupe hydroxy en position 3 ou 4 ou par un groupe hydroxyalkyle, diméthylaminoalkyle ou diéthylaminoalkyle dont la partie alkyle a 1 à 3 atomes de carbone.

**Patentansprüche** für die Vertragsstaaten: BE, CH, DE, FR, GB, IT, LI, LU, NL, SE.

1. Arzneimittel, enthaltend eine aktive Substanz und einen pharmazeutisch annehmbaren Träger, dadurch gekennzeichnet, daß die aktive Substanz eine Verbindung entsprechend der Formel

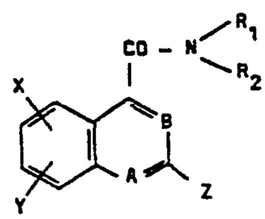

(I)

ist, worin bedeuten

$R_1$ eine gerade oder verzweigte Alkylgruppe mit 1 bis 6 Kohlenstoffatomen, eine Phenylgruppe, eine Cycloalkylgruppe mit 3 bis 6 Kohlenstoffatomen, eine Phenylalkyl- oder Cycloalkylalkylgruppe, deren Alkylteil 1 bis 3 Kohlenstoffatome trägt, oder eine Gruppe

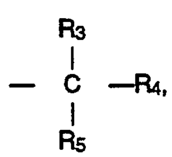

worin $R_3$ und $R_4$ Wasserstoffatome oder Alkylgruppen darstellen und $R_5$ eine Alkenyl- oder Alkinylgruppe ist, wobei die Summe der Kohlenstoffatome von $R_3$, $R_4$ und $R_5$ 2 bis 5 beträgt,

$R_2$ eine gerade oder verzweigte Alkylgruppe mit 1 bis 6 Kohlenstoffatomen, eine Phenylgruppe, eine Cycloalkylgruppe mit 3 bis 6 Kohlenstoffatomen, eine Phenylalkyl- oder Cycloalkylalkylgruppe, deren Alkylteil 1 bis 3 Kohlenstoffatome trägt, eine Gruppe

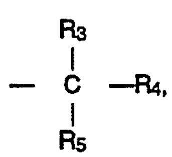

worin $R_3$, $R_4$ und $R_5$ wie vorstehend definiert sind, oder eine Gruppe $-(CH_2)_n-$ 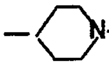 —H, worin n für 0, 1, 2 oder 3 steht,

wobei $R_1$ und $R_2$ darüber hinaus zusammen mit dem Stickstoffatom, an das sie gebunden sind, einen heterocyclischen Rest mit 5, 6 oder 7 Kettengliedern bilden können, der ein anderes Heteroatom, ausgewählt unter Stickstoff und Sauerstoff, enthalten kann und einen oder zwei Substituenten tragen kann, ausgewählt unter den Alkylgruppen mit 1 bis 3 Kohlenstoffatomen, der Hydroxygruppe, der Oxogruppe und den Hydroxyalkyl-, Dimethylaminoalkyl- und Diethylaminoalkylgruppen mit 1 bis 3 Kohlenstoffatomen im Alkylteil,

Z eine Phenyl-, Pyridyl-, Thienyl-, Thiazolyl-2-Gruppe oder eine Phenylgruppe, die durch einen oder zwei

Substituenten, ausgewählt unter den Halogenatomen, den Alkyl-, Alkoxy- und Alkylthiogruppen mit 1 bis 3 Kohlenstoffatomen, der Trifluormethylgruppe und der Nitrogruppe, substituiert ist,

X und Y sind identisch oder verschieden und bedeuten Wasserstoff- oder Halogenatome, Alkyl- oder Alkoxygruppen mit 1 bis 3 Kohlenstoffatomen, Nitro- oder Trifluormethylgruppen,

A bedeutet ein Stickstoffatom oder eine CH-Gruppe und

B bedeutet ein Stickstoffatom oder eine Gruppe CH mit Ausnahme des N,N-Diethyl-2-phenylchinolin-4-carboxamids,

oder ein Gemisch der stereoisomeren Verbindungen entsprechend der Formel (I) oder ein Salz einer solchen Verbindung oder ein Gemisch der stereoisomeren Verbindungen mit einer pharmazeutisch annehmbaren Säure.

2. Arzneimittel gemäß Anspruch 1, dadurch gekennzeichnet, daß in der Formel (I) X und Y Wasserstoffatome sind.

3. Arzneimittel gemäß einem der Ansprüche 1 oder 2, dadurch gekennzeichnet, daß in der Formel (I) 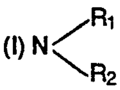

einen Rest Pyrrolidinyl, Piperidino, Morpholino, Piperazinyl, N-Methylpiperazinyl, 1,2,3,6-Tetrahydropyridyl, 2,3,4,5,6,7-Hexahydro-azepinyl, 4-Oxopiperidino, Piperidino, substituiert durch eine oder zwei Alkylgruppen mit 1 bis 3 Kohlenstoffatomen oder durch eine Hydroxygruppe in der 3- oder 4-Stellung oder durch eine Hydroxyalkyl-, Dimethylaminoalkyl- oder Diethylaminoalkylgruppe mit 1 bis 3 Kohlenstoffatomen im Alkylteil, bedeutet.

4. Arzneimittel gemäß Anspruch 2, dadurch gekennzeichnet, daß in Formel (I) X und Y Wasserstoffatome sind, A ein Stickstoffatom bedeutet, B ein Stickstoffatom oder eine Gruppe CH ist, Z die Phenylgruppe ist

und 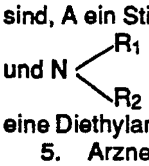

eine Diethylamino-, Piperidino- oder Pyrrolidinogruppe ist.

5. Arzneimittel gemäß Anspruch 1, dadurch gekennzeichnet, daß die aktive Substanz das N,N-Diethyl-3-phenyl-naphthalin-1-carboxamid oder eine Verbindung der Formel (I) gemäß Anspruch 1, worin X und Y Wasserstoffatome, Z eine Phenyl-, 2-Chlorphenyl- oder 3-Chlorphenylgruppe und mindestens einer der Substituenten $R_1$ und $R_2$ eine Alkylgruppe mit 3 bis 6 Kohlenstoffatomen, die in α-Stellung in bezug auf das Stickstoffatom verzweigt ist, bedeuten, oder das Salz einer solchen Verbindung mit einer pharmazeutisch annehmbaren Säure ist.

6. Racemische oder stereoisomere Verbindungen entsprechend der Formel (I) gemäß Anspruch 1, worin entweder A und B Stickstoffatome sind und X, Y, Z, $R_1$ und $R_2$ die in Anspruch 1 angegebenen Bedeutungen haben, oder A ist eine Gruppe CH, B ist ein Stickstoffatom und X, Y, Z, $R_1$ und $R_2$ haben die in Anspruch 1 angegebenen Bedeutungen,

oder A und B sind Gruppen CH, $R_1$ ist eine gerade oder verzweigte $C_{2-6}$-Alkylgruppe, Phenyl, $C_{3-6}$-Cycloalkyl, Phenylalkyl oder Cycloalkylalkyl mit 1 bis 3 Kohlenstoffatomen im Alkylteil oder

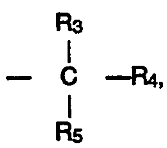

wobei $R_3$, $R_4$ und $R_5$ die in Anspruch 1 angegebenen Bedeutungen besitzen, $R_2$ ist eine gerade oder verzweigte $C_{1-6}$-Alkylgruppe, Phenyl, $C_{3-6}$-Cycloalkyl, Phenylalkyl oder Cycloalkylalkyl mit 1 bis 3 Kohlenstoffatomen im Alkylteil,

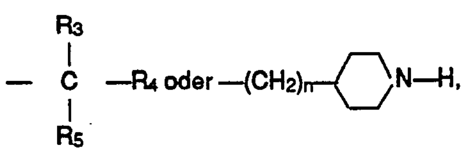

wobei $R_3$, $R_4$, $R_5$ und n die in Anspruch 1 angegebenen Bedeutungen haben, wobei $R_1$ und $R_2$ auch zusammen mit dem Stickstoffatom, an das sie gebunden sind, einen heterocyclischen Rest mit 5, 6 oder 7 Kettengliedern bilden können, der ein anderes Heteroatom, ausgewählt unter Stickstoff und Sauerstoff, enthalten kann und einen oder zwei Substituenten tragen kann, ausgewählt unter den $C_{1-3}$-Alkylgruppen, Hydroxy, Oxo, Hydroxyalkyl, Dimethylaminoalkyl und Diethylaminoalkyl mit 1 bis 3 Kohlenstoffatomen im Alkylteil, und Z, X und Y die in Anspruch 1 angegebenen Bedeutungen haben, oder A ist ein Stickstoffatom, B ist eine Gruppe CH, Z, X und Y haben die in Anspruch 1 angegebenen Bedeutungen, $R_1$ ist eine Phenyl-, $C_{3-6}$-Cycloalkyl-, Phenylalkyl- oder Cycloalkylalkylgruppe mit 1 bis 3 Kohlenstoffatomen im Alkylteil oder

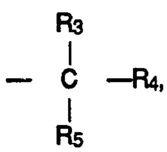

wobei $R_3$, $R_4$ und $R_5$ die in Anspruch 1 angegebenen Bedeutungen haben, $R_2$ ist eine Phenyl-, $C_{3-6}$-Cycloalkyl-, Phenylalkyl- oder Cycloalkylalkylgruppe mit 1 bis 3 Kohlenstoffatomen im Alkylteil,

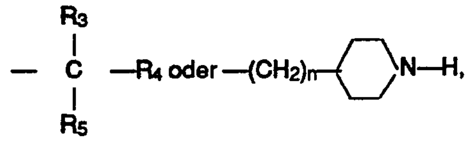

wobei $R_3$, $R_4$, $R_5$ und n die in Anspruch 1 angegebenen Bedeutungen haben, wobei $R_1$ und $R_2$ außerdem zusammen mit dem Stickstoffatom, an das sie gebunden sind, einen Rest Pyrrolidinyl, Piperidino, Morpholino, 1,2,3,6-Tetrahydro-pyridyl, 2,3,4,5,6,7-Hexahydroazepinyl, 4-Oxopiperidino oder Piperidino, substituiert durch eine oder zwei $C_{1-3}$-Alkylgruppen oder durch eine Hydroxygruppe in 3- oder 4-Stellung oder durch eine Hydroxyalkyl-, Dimethylaminoalkyl- oder Diethylaminoalkylgruppe mit 1 bis 3 Kohlenstoffatomen im Alkylteil, bilden können und $R_1$ und $R_2$ auch verschiedene $C_{1-6}$-Alkylgruppen sein können.

7. Verbindungen gemäß Anspruch 6, bei denen X und Y Wasserstoffatome sind.

8. Verbindungen gemäß Anspruch 6, worin entweder A eine Gruppe CH und B ein Stickstoffatom ist oder A und B Gruppen CH sind oder A und B Stickstoffatome sind und 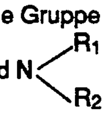 einen Rest Pyrrolidinyl, Piperidino, Morpholino, Piperazinyl, N-Methylpiperazinyl, 1,2,3,6-Tetrahydropyridyl, 2,3,4,5,6,7-Hexahydro-azepinyl, 4-Oxopiperidino, Piperidino, substituiert durch eine oder zwei Alkylgruppen mit 1 bis 3 Kohlenstoffatomen oder durch eine Hydroxygruppe in 3- oder 4-Stellung oder auch durch eine Hydroxyalkyl-, Dimethylaminoalkyl- oder Diethylaminoalkylgruppe mit 1 bis 3 Kohlenstoffatomen im Alkylteil, darstellt.

9. Verfahren zur Herstellung von Verbindungen gemäß Anspruch 6, dadurch gekennzeichnet, daß man ein Amin der Formel 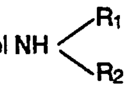
mit einer Verbindung der Formel

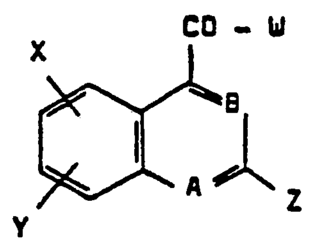

umsetzt, worin X, Y, Z, A, B, $R_1$ und $R_2$ in den obigen Formeln die gleichen Bedeutungen wie in Anspruch 6 haben und W eine Alkoxygruppe mit niedrigem Molekulargewicht, ein Chloratom oder eine Alkoxycarbonyloxygruppe mit niedrigem Molekulargewicht darstellt.

10. Verfahren zur Herstellung der Verbindungen gemäß Anspruch 6, worin $R_2$ eine Gruppe

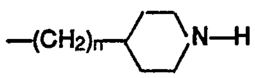

ist, dadurch gekennzeichnet, daß man ein Amin der Formel 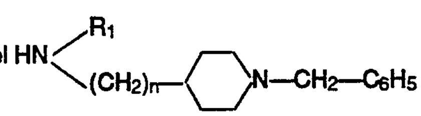

mit einer Verbindung der Formel

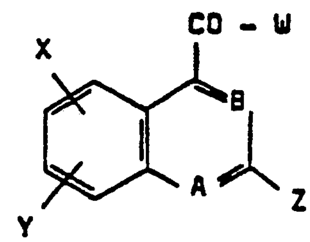

umsetzt, worin X, Y, Z, A, B, $R_1$ und n in den obigen Formeln die gleichen Bedeutungen wie in Anspruch 6 haben und W eine Alkoxygruppe mit niedrigem Molekulargewicht, ein Chloratom oder eine Alkoxycarbonyloxygruppe mit niedrigem Molekulargewicht darstellt, und daß man die so erhaltene Verbindung einer Debenzylierung unterwirft.

**Ansprüche** für den Vertragsstaat AT.

1. Verfahren zur Herstellung von racemischen oder stereoisomeren Verbindungen der Formel:

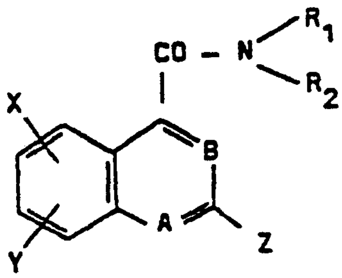

in welcher entweder A und B Stickstoffatome sind und $R_1$ eine geradkettige oder verzweigte Alkylgruppe mit 1 bis 6 Kohlenstoffatomen, eine Phenylgruppe, eine Cycloalkylgruppe mit 3 bis 6 Kohlenstoffatomen, eine Phenylalkyl- oder Cycloalkylalkylgruppe, deren Alkylteil 1 bis 3 Kohlenstoffatome enthält, oder eine Gruppe

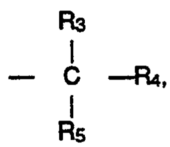

in welcher $R_3$ und $R_4$ Wasserstoffatome oder Alkylgruppen sind und $R_5$ eine Alkenyl- oder Alkinylgruppe ist, wobei die Summe der Kohlenstoffatome von $R_3$, $R_4$ und $R_5$ 2 bis 5 ist, darstellt, $R_2$ eine geradkettige oder verzweigte Alkylgruppe mit 1 bis 6 Kohlenstoffatomen, eine Phenylgruppe, eine Cycloalkylgruppe mit 3 bis 6 Kohlenstoffatomen, eine Phenylalkyl- oder Cycloalkylalkylgruppe, deren Alkylteil 1 bis 3 Kohlenstoffatome enthält, eine Gruppe

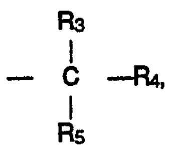

in welcher $R_3$, $R_4$ und $R_5$ die obige Bedeutung haben, oder eine Gruppe $—(CH_2)_n —$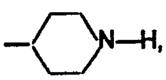,

in welcher n gleich 0, 1, 2 oder 3 ist, darstellt, wobei $R_1$ und $R_2$ auch zusammen mit dem Stickstoffatom, an das sie gebunden sind, einen heterocyclischen Rest mit 5, 6 oder 7 Gliedern bilden können, der ein weiteres Heteroatom, ausgewählt aus Stickstoff und Sauerstoff, enthalten kann und einen oder zwei Substituenten, ausgewählt aus den Alkylgruppen mit 1 bis 3 Kohlenstoffatomen, der Hydroxygruppe, der Oxogruppe und den Hydroxyalkyl-, Dimethylaminoalkyl- und Diäthylaminoalkylgruppen, deren Alkylteil 1 bis 3 Kohlenstoffatome enthält, tragen kann,

Z eine Phenyl-, Pyridyl-, Thienyl-, 2-Thiazolylgruppe oder eine durch einen oder zwei Substituenten, ausgewählt aus den Halogenatomen, den Alkyl-, Alkoxy- und Alkylthiogruppen mit 1 bis 3 Kohlenstoffatomen, der Trifluormethylgruppe und der Nitrogruppe, substituierte Phenylgruppe darstellt,

X und Y gleich oder verschieden sind und Wasserstoff- oder Halogenatome, Alkyl- oder Alkoxygruppen mit 1 bis 3 Kohlenstoffatomen, Nitro- oder Trifluormethylgruppen darstellen, oder A eine CH-Gruppe ist, B ein Stickstoffatom ist und X, Y, Z, $R_1$ und $R_2$ die obigen Bedeutungen haben;

oder A und B CH-Gruppen sind, $R_1$ eine geradkettige oder verzweigte $C_{2-6}$-Alkyl-, Phenyl-, $C_{3-6}$-Cycloalkyl-, Phenylalkyl- oder Cycloalkylalkylgruppe, deren Alkylteil 1 bis 3 Kohlenstoffatome enthält, oder

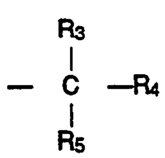

ist, wobei $R_3$, $R_4$ und $R_5$ die obigen Bedeutungen haben,

$R_2$ eine geradkettige oder verzweigte $C_{1-6}$-Alkyl-, Phenyl-, $C_{3-6}$-Cycloalkyl-, Phenylalkyl- oder Cycloalkylalkylgruppe, deren Alkylteil 1 bis 3 Kohlenstoffatome enthält,

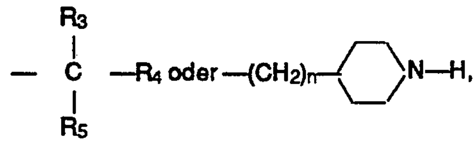

ist, wobei $R_3$, $R_4$, $R_5$ und n die obigen Bedeutungen haben ist, und $R_1$ und $R_2$ außerdem zusammen mit dem Stickstoffatom, an das sie gebunden sind, einen heterocyclischen Rest mit 5, 6 oder 7 Gliedern bilden können, der ein weiteres Heteroatom, ausgewählt aus Stickstoff und Sauerstoff, enthalten kann und einen oder zwei Substituenten, ausgewählt aus den $C_{1-3}$-Alkyl-, Hydroxy-, Oxo-, Hydroxyalkyl-, Dimethylaminoalkyl- und Diäthylaminoalkylgruppen, deren Alkylteil 1 bis 3 Kohlenstoffatome enthält, tragen kann, und Z, X und Y die obigen Bedeutungen haben; oder A ein Stickstoffatom ist, B eine CH-Gruppe ist, Z, X und Y die obigen Bedeutungen haben, $R_1$ eine Phenyl-, $C_{3-6}$-Cycloalkyl-, Phenylalkyl- oder Cycloalkylalkylgruppe, deren Alkylteil 1 bis 3 Kohlenstoffatome enthält, oder

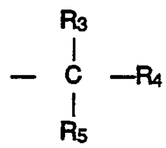

ist, wobei $R_3$, $R_4$ und $R_5$ die obigen Bedeutungen haben, $R_2$ eine Phenyl-, $C_{3-6}$-Cycloalkyl-, Phenylalkyl- oder Cycloalkylalkylgruppe, deren Alkylteil 1 bis 3 Kohlenstoffatome enthält

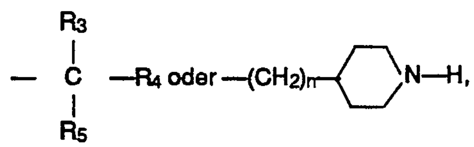

ist, wobei $R_3$, $R_4$, $R_5$ und n die obigen Bedeutungen haben und $R_1$ und $R_2$ außerdem zusammen mit dem Stickstoffatom, an das sie gebunden sind, einen Pyrrolidinyl-, Piperidino-, Morpholino-, 1,2,3,6-Tetrahydro- pyridyl-, 2,3,4,5,6,7-Hexahydro-azepinyl-, 4-Oxopiperidinorest oder einen Piperidinorest, substituiert durch eine oder zwei $C_{1-3}$-Alkylgruppen oder durch eine Hydroxygruppe in 3-oder 4-Stellung oder durch eine Hydroxyalkyl-, Dimethylaminoalkyl- oder Diäthylaminoalkylgruppe, deren Alkylteil 1 bis 3 Kohlenstoffatome enthält, bilden können oder $R_1$ und $R_2$ verschiedene $C_{1-6}$-Alkylgruppen sein können, dadurch gekennzeichnet, daß man ein Amin

der Formel 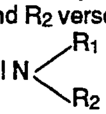

mit einer Verbindung der Formel:

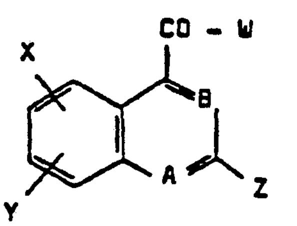

worin X, Y, Z, A, B, $R_1$ und $R_2$ die obigen Bedeutungen haben und W eine Alkoxygruppe mit niedrigem Molekulargewicht, ein Chloratom oder eine Alkoxycarbonyloxygruppe mit niedrigem Molekulargewicht darstellt, reagieren läßt.

2. Verfahren nach Anspruch 1 zur Herstellung von Verbindungen der Formel (I), in welcher $R_2$ eine Gruppe

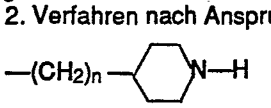

ist, dadurch gekennzeichnet, daß man ein Amin der Formel:

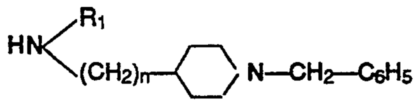

mit einer Verbindung der Formel:

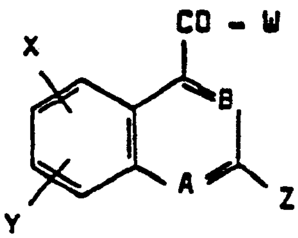

reagieren läßt, wobei X, Y, Z, A, B, $R_1$ und n in den obigen Formeln die gleiche Bedeutung wie im Anspruch 1 haben und W eine Alkoxygruppe mit niedrigem Molekulargewicht, ein Chloratom oder eine Alkoxycarbonyloxygruppe mit niedrigem Molekulargewicht darstellt, und die so erhaltene Verbindung einer Debenzylierung unterwirft.

3. Verfahren nach einem der Ansprüche 1 oder 2 zur Herstellung von Verbindungen der Formel (I), in welcher X und Y Wasserstoffatome sind.

4. Verfahren nach Anspruch 1 zur Herstellung von Verbindungen der Formel (I), in welcher X und Y Wasserstoffatome sind, A ein Stickstoffatom ist, B ein Stickstoffatom oder eine CH-Gruppe ist, Z die Phenylgruppe ist und 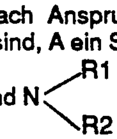

eine Diäthylamino-, Piperidino- oder Pyrrolidinogruppe ist.

5. Verfahren nach Anspruch 1 zur Herstellung von Verbindungen der Formel (I), in welcher X und Y Wasserstoffatome sind, Z eine Phenyl-, 2- oder 3-Chlorphenylgruppe ist und zumindest einer der Substituenten $R_1$ und $R_2$ eine Alkylgruppe mit 3 bis 6 Kohlenstoffatomen, verzweigt in α-Position in bezug auf das Stickstoffatom, ist und A und B die in Anspruch 1 angegebene Bedeutung haben.

6. Verfahren nach Anspruch 1 zur Herstellung von Verbindungen der Formel (I), in welcher entweder A eine CH-Gruppe und B ein Stickstoffatom ist oder A und B CH-Gruppen sind oder A und B Stickstoffatome sind und 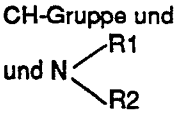

einen Pyrrolidinyl-, Piperidino-, Morpholino-, Piperazinyl-, N-Methylpiperazinyl-, 1,2,3,6-Tetrahydro-pyridyl-, 2,3,4,5,6,7-Hexahydro-azepinyl-, 4-Oxo-piperidinorest oder einen Piperidinorest, substituiert durch eine oder zwei Alkylgruppen mit 1 bis 3 Kohlenstoffatomen oder durch eine Hydroxygruppe in 3- oder 4-Stellung oder durch eine Hydroxyalkyl-, Dimethylaminoalkyl- oder Diäthylaminoalkylgruppe, deren Alkylteil 1 bis 3 Kohlenstoffatome enthält, darstellt.

**Claims** for the contracting States BE, CH, DE, FR, GB, IT, LI, LU, NL, SE.

1. Medications containing an active substance and a pharmaceutically acceptable vehicle, characterized in that the active substance is a compound corresponding to the formula:

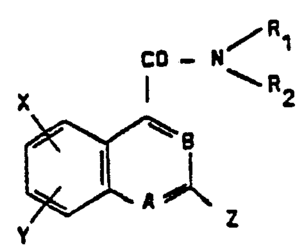

(I)

in which $R_1$ denotes a straight-chain or branched alkyl group containing 1 to 6 carbon atoms, a phenyl group, a cycloalkyl group containing 3 to 6 carbon atoms, a phenylalkyl or cycloalkylalkyl group in which the alkyl moiety contains 1 to 3 carbon atoms, or a group

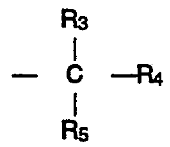

in which $R_3$ and $R_4$ are hydrogen atoms or alkyl groups and $R_5$ is an alkenyl or alkynyl group, the sum of the carbon atoms in $R_3$, $R_4$ and $R_5$ being from 2 to 5, $R_2$ denotes a straight-chain or branched alkyl group containing 1 to 6 carbon atoms, a phenyl group, a cycloalkyl group containing 3 to 6 carbon atoms, a phenylalkyl or cycloalkylalkyl group in which the alkyl moiety contains 1 to 3 carbon atoms, a group

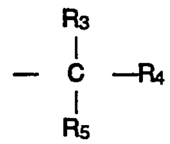

in which $R_3$, $R_4$ and $R_5$ are such as defined above, or a group —$(CH_2)_n$— 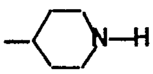

in which n is 0, 1, 2 or 3, it being furthermore possible for $R_1$ and $R_2$ to form together with the nitrogen atom to which they are attached a 5-, 6- or 7-membered heterocyclic radical capable of containing another hetero atom chosen from nitrogen and oxygen and capable of bearing one or two substituents chosen from alkyl groups containing 1 to 3 carbon atoms, the hydroxyl group, the oxo group and the hydroxyalkyl, dimethylaminoalkyl and diethylaminoalkyl groups in which the alkyl moiety contains 1 to 3 carbon atoms,

Z denotes a phenyl, pyridyl, thienyl or 2-thiazolyl group, or a phenyl group substituted by one or two substituents chosen from halogen atoms, alkyl, alkoxy and alkylthio groups containing 1 to 3 carbon atoms, the trifluoromethyl group and the nitro group,

X and Y are identical or different and denote hydrogen or halogen atoms, alkyl or alkoxy groups containing 1 to 3 carbon atoms, or nitro or trifluoromethyl groups,

A denotes a nitrogen atom or a CH group and

B denotes a nitrogen atom or a CH group, with the exception of N,N-diethyl-2-phenylquinoline-4-carboxamide,

or a mixture of stereoisomeric compounds corresponding to the formula (I), or a salt of a compound or mixture of stereoisomeric compounds of this kind with a pharmaceutically acceptable acid.

2. Medications according to Claim 1, characterized in that, in the formula (I), X and Y are hydrogen atoms.

3. Medications according to either of Claims 1 or 2, characterized in that, in the formula (I) 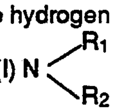
denotes a pyrrolidinyl, piperidino, morpholino, piperazinyl, N-methylpiperazinyl, 1,2,3,6-tetrahydropyridyl, 2,3,4,5,6,7-hexahydroazepinyl, 4-oxopiperidino or piperidino radical substituted by one or two alkyl groups containing 1 to 3 carbon atoms or by a hydroxyl group in the 3 or 4 position or alternatively by a hydroxyalkyl, dimethylaminoalkyl or diethylaminoalkyl group in which the alkyl moiety contains 1 to 3 carbon atoms.

4. Medications according to Claim 2, characterized in that, in the formula (I), X and Y are hydrogen atoms, A is a nitrogen atom, B is a nitrogen atom or a CH group, Z is the phenyl group and 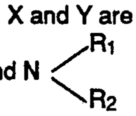
is a diethylamino, piperidino or pyrrolidino group.

5. Medications according to Claim 1, characterized in that the active substance is N,N-diethyl-3-phenylnaphthalene-1-carboxamide or a compound of formula (I) according to Claim 1, in which X and Y are hydrogen atoms, Z is a phenyl, 2-chlorophenyl or 3-chlorophenyl group, and at least one of the substituents $R_1$ and $R_2$ is an alkyl group containing 3 to 6 carbon atoms which is branched in the $\alpha$ position with respect to the nitrogen atom, or a salt of a compound of this kind with a pharmaceutically acceptable acid.

6. Racemic or stereoisomeric compounds corresponding to the formula (I) according to Claim 1, in which either A and B are nitrogen atoms and X, Y, Z, $R_1$ and $R_2$ have the meanings indicated in Claim 1,

or A is a CH group, B is a nitrogen atom and X, Y, Z, $R_1$ and $R_2$ have the meanings indicated in Claim 1, or A and B are CH groups, $R_1$ is a $C_{2-6}$ straight-chain or branched alkyl, phenyl or $C_{3-6}$ cycloalkyl group or a phenylalkyl or cycloalkylalkyl group in which the alkyl moiety contains 1 to 3 carbon atoms or

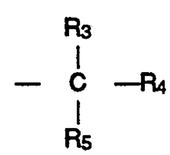

$R_3$, $R_4$ and $R_5$ having the meanings indicated in Claim 1, $R_2$ is a $C_{1-6}$ straight-chain or branched alkyl, phenyl, $C_{3-6}$ cycloalkyl group or a phenylalkyl or cycloalkylalkyl group in which the alkyl moiety contains 1 to 3 carbon atoms,

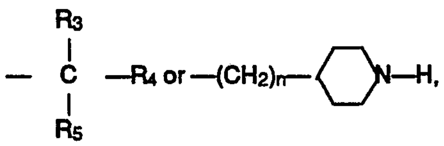

$R_3$, $R_4$, $R_5$ and n having the meanings indicated in Claim 1, it being furthermore possible for $R_1$ and $R_2$ to form together with the nitrogen atom to which they are attached a 5-, 6- or 7-membered heterocyclic radical capable of containing another hetero atom chosen from nitrogen and oxygen and capable of bearing one or two substituents chosen from $C_{1-3}$ alkyl, hydroxyl, oxo, hydroxyalkyl, dimethylaminoalkyl and diethylaminoalkyl groups in which the alkyl moiety contains from 1 to 3 carbon atoms, and Z, X and Y have the meanings indicated in Claim 1, or A is a nitrogen atom, B is a CH group, Z, X and Y have the meanings indicated in Claim 1, $R_1$ is a phenyl or $C_{3-6}$ cycloalkyl group or a phenylalkyl or cycloalkylalkyl group in which the alkyl moiety contains 1 to 3 carbon atoms, or

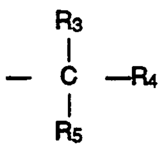

$R_3$, $R_4$ and $R_5$ having the meanings indicated in Claim 1, $R_2$ is a phenyl, $C_{3-6}$ cycloalkyl group or a phenylalkyl or cycloalkylalkyl group in which the alkyl moiety contains 1 to 3 carbon atoms,

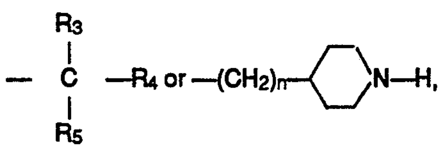

$R_3$, $R_4$, $R_5$ and n having the meanings indicated in Claim 1, it being furthermore possible for $R_1$ and $R_2$ to form together with the nitrogen atom to which they are attached a pyrrolidinyl, piperidino, morpholino, 1,2,3,6-tetrahydropyridyl, 2,3,4,5,6,7-hexahydroazepinyl, 4-oxopiperidino or piperidino radical substituted by one or two $C_{1-3}$ alkyl group or by a hydroxyl group in the 3 or 4 position or by a hydroxyalkyl, dimethylaminoalkyl or diethylaminoalkyl group in which the alkyl moiety contains from 1 to 3 carbon atoms,

$R_1$ and $R_2$ may also be different $C_{1-6}$ alkyl groups.

7. Compounds according to Claim 6 in which X and Y are hydrogen atoms.

8. Compounds according to Claim 6 in which either A is a CH group and B is a nitrogen atom, or A and B are CH groups, or A and B are nitrogen atoms and 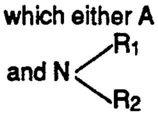 denotes a pyrrolidinyl, piperidino, morpholino, piperazinyl, N-methylpiperazinyl, 1,2,3,6-tetrahydropyridyl, 2,3,4,5,6,7-hexahydroazepinyl, 4-oxopiperidino or piperidino radical substituted by one or two alkyl groups containing 1 to 3 carbon atoms or by a hydroxyl group in the 3 or 4 position or alternatively by a hydroxyalkyl, dimethylaminoalkyl or diethylaminoalkyl group in which the alkyl moiety contains 1 to 3 carbon atoms.

9. Process for the preparation of the compounds according to Claim 6, characterized in that an amine of formula 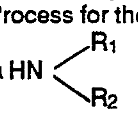 is reacted with a compound of formula:

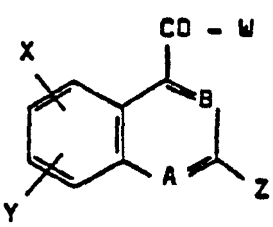

X, Y, Z, A, B, $R_1$ and $R_2$ having the same meanings in the above formulae as in Claim 6 and W denoting an alkoxy group of low molecular weight, a chlorine atom or an alkoxycarbonyloxy group of low molecular weight.

10. Process for the preparation of the compounds according to Claim 6 in which $R_2$ is a group

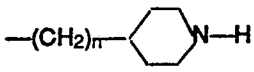

characterized in that an amine of formula: 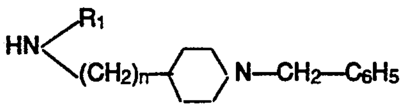

is reacted with a compound of formula:

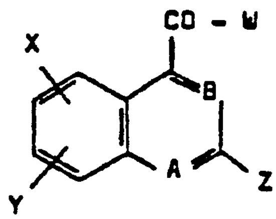

X, Y, Z, A, B, $R_1$ and n having the same meanings in the above formulae as in Claim 6 and W denoting an alkoxy group of low molecular weight, a chlorine atom or an alkoxycarbonyloxy group of low molecular weight, and the compound thus obtained is subjected to a debenzylation.

**Claims** for the contracting State AT.

1. Process for the preparation of racemic or stereoisomeric compounds of formula:

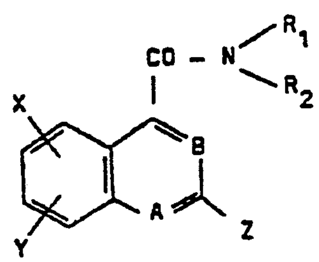

(I)

in which either A and B are nitrogen atoms and $R_1$ denotes a straight-chain or branched alkyl group containing 1 to 6 carbon atoms, a phenyl group, a cycloalkyl group containing 3 to 6 carbon atoms, a phenylalkyl or cycloalkylalkyl group in which the alkyl moiety contains 1 to 3 carbon atoms, or a group

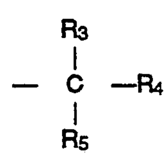

in which $R_3$ and $R_4$ are hydrogen atoms or alkyl groups and $R_5$ is an alkenyl or alkynyl group, the sum of the carbon atoms in $R_3$, $R_4$ and $R_5$ being from 2 to 5, $R_2$ denotes a straight-chain or branched alkyl group containing 1 to 6 carbon atoms, a phenyl group, a cycloalkyl group containing 3 to 6 carbon atoms, a phenylalkyl or cycloalkylalkyl group in which the alkyl moiety contains 1 to 3 carbon atoms, a group

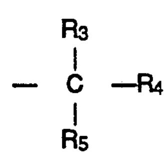

in which $R_3$, $R_4$ and $R_5$ are such as defined above, or a group 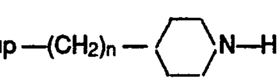

in which n is 0, 1, 2 or 3, it being furthermore possible for $R_1$ and $R_2$ to form together with the nitrogen atom to

which they are attached a 5-, 6- or 7-membered heterocyclic radical capable of containing another hetero atom chosen from nitrogen and oxygen and capable of bearing one or two substituents chosen from alkyl groups containing 1 to 3 carbon atoms, the hydroxyl group, the oxo group and the hydroxyalkyl, dimethylaminoalkyl and diethylaminoalkyl groups in which the alkyl moiety contains 1 to 3 carbon atoms,

Z denotes a phenyl, pyridyl, thienyl, 2-thiazolyl group or a phenyl group substituted by one or two substituents chosen from halogen atoms, alkyl, alkoxy and alkylthio groups containing 1 to 3 carbon atoms, the trifluoromethyl group and the nitro group,

X and Y are identical or different and denote hydrogen or halogen atoms, alkyl or alkoxy groups containing 1 to 3 carbon atoms, or nitro or trifluoromethyl groups,

either A is a CH group, B is a nitrogen atom and X, Y, Z, $R_1$ and $R_2$ have the meanings given above; or A and B are CH groups, $R_1$ is a $C_{2-6}$ straight-chain or branched alkyl, phenyl or $C_{3-6}$ cycloalkyl group or a phenylalkyl or cycloalkylalkyl group in which the alkyl moiety contains 1 to 3 carbon atoms, or

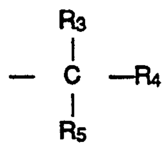

$R_3$, $R_4$ and $R_5$ having the meanings indicated above, $R_2$ is a $C_{1-6}$ straight-chain or branched alkyl, phenyl, $C_{3-6}$ cycloalkyl group or a phenylalkyl or cycloalkylalkyl group in which the alkyl moiety contains 1 to 3 carbon atoms,

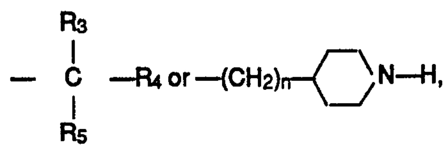

$R_3$, $R_4$, $R_5$ and n having the meanings indicated above, $R_1$ and $R_2$ being furthermore capable of forming together with the nitrogen atom to which they are attached a 5-, 6- or 7-membered heterocyclic radical capable of containing another hetero atom chosen from nitrogen and oxygen and capable of bearing one or two substituents chosen from $c_{1-3}$ alkyl, hydroxyl, oxo, hydroxyalkyl, dimethylaminoalkyl and diethylaminoalkyl groups in which the alkyl moiety contains 1 to 3 carbon atoms, and Z,X, and Y have the meanings indicated above;

or A is a nitrogen atom, B is a CH group, Z, X and Y have the meanings indicated above, $R_1$ is a phenyl or $C_{3-6}$ cycloalkyl group or a phenylalkyl or cycloalkylalkyl group in which the alkyl moiety contains 1 to 3 carbon atoms, or

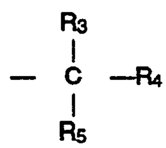

$R_3$, $R_4$ and $R_5$ having the meanings indicated above, $R_2$ is a phenyl or $C_{3-6}$ cycloalkyl group or a phenylalkyl or cycloalkylalkyl group in which the alkyl moiety contains 1 to 3 carbon atoms,

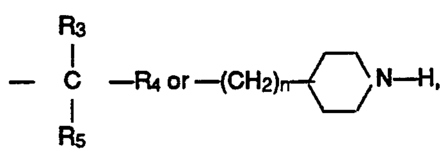

$R_3$, $R_4$, $R_5$ and n having the meanings indicated above, it being furthermore possible for $R_1$ and $R_2$ to form together with the nitrogen atom to which they are attached a pyrrolidinyl, piperidino, morpholino, 1,2,3,6-tetrahydropyridyl, 2,3,4,5,6,7-hexahydroazepinyl, 4-oxopiperidino or piperidino radical substituted by one or two $C_{1-3}$ alkyl groups or by a hydroxyl group in the 3 or 4 position or by a hydroxyalkyl, dimethylaminoalkyl or diethylaminoalkyl group in which the alkyl moiety contains 1 to 3 carbon atoms, or $R_1$ and $R_2$ may be different $C_{1-6}$ alkyl groups, characterized in that an amine of formula 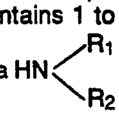 is reacted with a compound of formula:

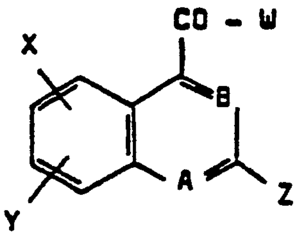

X, Y, Z, A, B, $R_1$ and $R_2$ having the same meanings as above and W denoting an alkoxy group of low molecular weight, a chlorine atom or an alkoxycarbonyloxy group of low molecular weight.

2. Process according to Claim 1 for the preparation of the compounds of formula (I) in which $R_2$ is a group

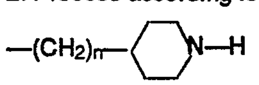

characterized in that an amine of formula: 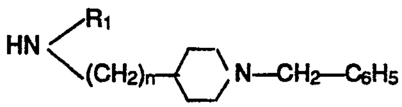

is reacted with a compound of formula:

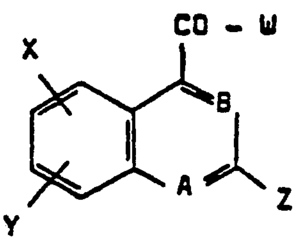

X, Y, Z, A, B, $R_1$ and n having the same meanings in the above formulae as in Claim 1, and W denoting an alkoxy group of low molecular weight, a chlorine atom or an alkoxycarbonyloxy group of low molecular weight, and the compound thus obtained is subjected to a debenzylation.

3. Process according to either of Claims 1 and 2 for the preparation of compounds of formula (I) in which X and Y are hydrogen atoms.

4. Process according to Claim 1 for the preparation of compounds of formula (I) in which X and Y are hydrogen atoms, A is a nitrogen atom, B is a nitrogen atom or a CH group, Z is the phenyl group and 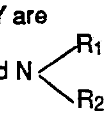 is a diethylamino, piperidino or pyrrolidino group.

5. Process according to Claim 1 for the preparation of compounds of formula (I) in which X and Y are hydrogen atoms, Z is a phenyl, 2- or 3-chlorophenyl group and at least one of the substituents $R_1$ and $R_2$ is an alkyl group containing 3 to 6 carbon atoms which is branched in the α position with respect to the nitrogen atom, and A and B are as defined in Claim 1.

6. Process according to Claim 1 for the preparation of the compounds of formula (I) in which either A is a CH group and B is a nitrogen atom, or A and B are CH groups, or A and B are nitrogen atoms and 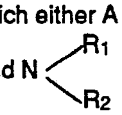 denotes a pyrrolidinyl, piperidino, morpholino, piperazinyl, N-methylpiperazinyl, 1,2,3,6-tetrahydropyridyl, 2,3,4,5,6,7-hexahydroazepinyl, 4-oxopiperidino, or piperidino radical substituted by one or two alkyl groups containing 1 to 3 carbon atoms or by a hydroxyl group in the 3 or 4 position or by a hydroxyalkyl, dimethylaminoalkyl or diethylaminoalkyl group in which the alkyl moiety contains 1 to 3 carbon atoms.